(19) Europäisches Patentamt
European Patent Office
Office européen des brevets

(11) Publication number: 0 211 220 A1

(12) # EUROPEAN PATENT APPLICATION

(21) Application number: 86108736.9

(22) Date of filing: 26.06.86

(51) Int. Cl.⁴: C 07 F 9/65
A 61 K 31/675, C 07 D 487/04
//(C07D487/04, 243:00, 237:00)

(30) Priority: 01.07.85 GB 8516604
18.04.86 GB 8609591

(43) Date of publication of application: 25.02.87 Bulletin 87/9

(84) Designated Contracting States: AT BE CH DE FR GB IT LI LU NL SE

(71) Applicant: F. HOFFMANN-LA ROCHE & CO. Aktiengesellschaft

CH-4002 Basel(CH)

(72) Inventor: Lawton, Geoffrey
109 Whitehill Road
Hitchin Herts(GB)

(72) Inventor: Redshaw, Sally
29 Hertford Road
Stevenage Herts(GB)

(74) Representative: Lederer, Franz, Dr. et al,
Vanderwerth, Lederer & Riederer Patentanwälte
Lucile-Grahnstrasse 22
D-8000 München 80(DE)

(54) Pyridazo]1,2-a[]1,2[diazepine derivatives, intermediate products, process for their preparation and medicaments containing them.

(57) Verbindungen der Formel

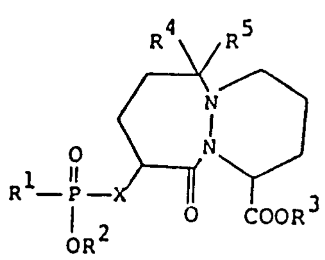

I

worin $R^1$ Hydroxy, Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy oder Aralkoxy, $R^2$ und $R^3$ je Wasserstoff, Alkyl oder Aralkyl, $R^4$ und $R^5$ je Wasserstoff oder zusammen eine Oxogruppe und X ein Sauerstoffatom oder die Gruppe $-NR^6-$ oder $-(CH_2)_n-$ bedeuten, worin $R^6$ Wasserstoff, Alkyl oder Aralkyl und n 0, 1 oder 2 bedeuten,
und pharmazeutisch verwendbare Salze davon besitzen antihypertensive Eigenschaften und können demnach in Form pharmazeutischer Präparate verwendet werden. Sie können nach bekannten Methoden hergestellt werden.



Croydon Printing Company Ltd

F. HOFFMANN-LA ROCHE & CO. Aktiengesellschaft, Basel/Schweiz

RAN 4019/97

# Pyridazo[1,2-a][1,2]diazepinderivate, Zwischenprodukte und Verfahren zu deren Herstellung sowie diese enthaltende Arzneimittel

Die vorliegende Erfindung betrifft Pyridazo[1,2-a][1,2]-diazepinderivate, ein Verfahren zu deren Herstellung sowie Arzneimittel enthaltend diese Derivate.

Die erfindungsgemässen Pyridazo[1,2-a][1,2]diazepinderivate sind Verbindungen der allgemeinen Formel

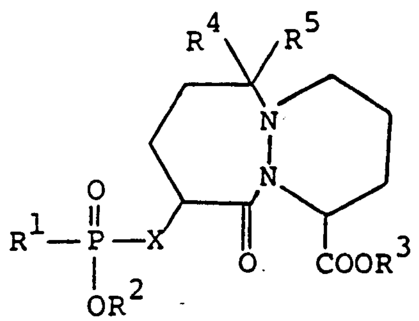

I

worin $R^1$ Hydroxy, Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy

oder Aralkoxy, $R^2$ und $R^3$ je Wasserstoff, Alkyl oder Aralkyl, $R^4$ und $R^5$ je Wasserstoff oder zusammen eine Oxogruppe und X ein Sauerstoffatom oder die Gruppe $-NR^6-$ oder $-(CH_2)_n-$ bedeuten, worin $R^6$ Wasserstoff, Alkyl oder Aralkyl und n 0, 1 oder 2 bedeuten, und pharmazeutisch verwendbare Salze davon.

Die Verbindungen der Formel I enthalten zwei asymmetrische Kohlenstoffatome und, falls $R^1$ und $OR^2$ unterschiedliche Bedeutungen haben, ein chirales Phosphoratom. Die vorliegende Erfindung umfasst nicht nur die optisch einheitlichen Formen dieser Verbindungen, sondern auch die verschiedenen diastereoisomeren Racemate und Mischungen der verschiedenen diastereoisomeren Racemate.

Der in der vorliegenden Beschreibung verwendete Ausdruck "Alkyl" - allein oder in Kombination - betrifft geradkettige oder verzweigte Alkylreste mit 1-8, vorzugsweise 1-4, Kohlenstoffatomen, z.B. Methyl, Aethyl, Propyl, Isopropyl, Butyl, tert.Butyl, Pentyl, Hexyl und dergleichen. Der Ausdruck "Aralkyl" betrifft Alkylgruppen wie sie oben definiert sind, in denen eines der Wasserstoffatome durch Phenyl oder durch Halogen, Alkyl, Alkoxy, Trifluormethyl, Nitro, Amino, Iminoalkylamino und dergleichen ein- oder mehrfach substituiertes Phenyl ersetzt ist. Beispiele von Aralkylgruppen sind Benzyl, Phenäthyl, 3-Phenylpropyl, 4-Chlorbenzyl, 4-Methoxybenzyl, 3-(4-Chlorphenyl)propyl, 3-(4-Methoxyphenyl)propyl, 4-Nitrophenäthyl, 4-Aminophenäthyl, 4-(1--Iminoäthylamino)phenäthyl und dergleichen. Der Ausdruck "Halogen" betrifft die vier Halogene Fluor, Chlor, Brom und Jod. Beispiele von Hydroxyalkylgruppen sind Hydroxymethyl, 2-Hydroxyäthyl und dergleichen. Aminomethyl, 2-Aminoäthyl und dergleichen sind Beispiele für Aminoalkylgruppen. Methylaminomethyl, 2-Methylaminoäthyl und 2-Aethylaminoäthyl sind Beispiele von Monoalkylaminoalkylgruppen und 2-Dimethylaminoäthyl, 2-Diäthylaminoäthyl und 3-Dimethylaminopropyl sind Beispiele von Dialkylaminoalkylgruppen. Der

Acylrest einer Acylaminoalkylgruppe leitet sich von einer gesättigten oder ungesättigten aliphatischen Carbonsäure, einer cycloaliphatischen Carbonsäure, einer aromatischen Carbonsäure, einer araliphatischen Carbonsäure oder einer heterocyclischen Carbonsäure ab, z.B. Essigsäure, Propionsäure, Buttersäure, Valeriansäure, Cyclopropancarbonsäure, Cyclopentancarbonsäure, Benzoesäure, p-Chlorbenzoesäure, Phenylessigsäure, Nikotinsäure und dergleichen. Der Ausdruck "Haloalkyl" betrifft eine Alkylgruppe mit einem Halogensubstituenten, beispielsweise Chlormethyl, 2-Chloräthyl, 3-Brompropyl und dergleichen. Beispiele von Carboxyalkylgruppen sind Carboxymethyl, 2-Carboxyäthyl und dergleichen. Der Ausdruck "Alkoxy" - allein oder in Kombination wie in Alkoxycarbonyl - betrifft eine Alkylgruppe wie oben definiert, welche über ein Sauerstoffatom gebunden ist. Beispiele von Alkoxygruppen sind Methoxy, Aethoxy, Propoxy, Isopropoxy, Butoxy, tert.Butoxy, Pentyloxy, Hexyloxy und dergleichen. Beispiele von Alkoxycarbonylaminoalkylgruppen sind Methoxycarbonylaminomethyl, 2-Aethoxycarbonylaminoäthyl, 3-Aethoxycarbonylaminopropyl und dergleichen. Methoxycarbonylmethyl, 2-Methoxycarbonyläthyl, 3-Aethoxycarbonylpropyl und dergleichen sind Beispiele für Alkoxycarbonylalkylgruppen. Der Ausdruck "Aralkoxy" betrifft eine Aralkylgruppe wie oben definiert, welche über ein Sauerstoffatom gebunden ist. Beispiele von Aralkoxygruppen sind Benzyloxy, 4-Chlorbenzyloxy, 2-Phenyläthoxy, 3-Phenylpropoxy und dergleichen.

Die Verbindungen der Formel I, die sauer sind, bilden pharmazeutisch verwendbare Salze mit Basen. Beispiele solcher Salze sind Alkalimetallsalze, z.B. Natrium- und Kaliumsalze, Erdalkalimetallsalze, z.B. Calcium- und Magnesiumsalze, Ammoniumsalze und Salze mit organischen Aminen, z.B. Dicyclohexylaminsalze. Die Verbindungen der Formel I, die basisch sind, bilden pharmazeutisch verwendbare Salze mit Säuren. Beispiele solcher Salze sind Mineralsäuresalze, wie Hydrohalogenide, z.B. Hydrobromide, Hydrochloride und

dergleichen, Sulfate, Phosphate, Nitrate und dergleichen, und Salze mit organischen Säuren, wie Acetate, Maleate, Fumarate, Tartrate, Citrate, Salicylate, Methansulfonate, p-Toluolsulfonate und dergleichen.

$R^1$ in Formel I bedeutet vorzugsweise Hydroxy, Alkyl, Aralkyl oder Alkoxy. $R^2$ bedeutet bevorzugt Wasserstoff. Vorzugsweise bedeutet $R^3$ Wasserstoff. X bedeutet vorzugsweise ein Sauerstoffatom oder die Gruppe -NH- oder $-(CH_2)_n-$, worin n 0 oder 1 bedeutet.

Aus dem obigen folgt, dass besonders bevorzugte Verbindungen der Formel I solche sind, worin $R^1$ Hydroxy, Alkyl, Aralkyl oder Alkoxy, $R^2$ Wasserstoff, $R^3$ Wasserstoff und X ein Sauerstoffatom oder die Gruppe -NH- oder $-(CH_2)_n-$ bedeuten, worin n 0 oder 1 bedeutet.

Ganz besonders bevorzugte Verbindungen der Formel I sind:

Octahydro-9(S)-phosphonomethyl-10-oxo-6H-pyridazo[1,2-a][1,2]diazepin-1(S)-carbonsäure,

Octahydro-9(S)-[[(hydroxy)methylphosphinyl]methyl]-6,10-dioxo-6H-pyridazo[1,2-a][1,2]diazepin-1(S)-carbonsäure,

Octahydro-9(S)-phosphono-6,10-dioxo-6H-pyridazo[1,2-a][1,2]diazepin-1(S)-carbonsäure,

9(S)-Diäthoxyphosphinyl-octahydro-6,10-dioxo-6H-pyridazo[1,2-a][1,2]diazepin-1(S)-carbonsäure,

Octahydro-9(S)-[[hydroxy(methoxy)phosphinyl]methyl]-6,10-dioxo-6H-pyridazo[1,2-a][1,2]diazepin-1(S)-carbonsäure,

Octahydro-9(S)-phosphonomethyl-6,10-dioxo-6H-pyridazo-[1,2-a][1,2]diazepin-1(S)-carbonsäure,

9(S)-[[Hydroxy(3-phenylpropyl)phosphinyl)]methyl]-octahydro-6,10-dioxo-6H-pyridazo[1,2-a][1,2]diazepin-1(S)-carbonsäure,

9(S)-[[Hydroxy(phenäthyl)phosphinyl]amino]-octahydro-10-oxo-6H-pyridazo[1,2-a][1,2]diazepin-1(S)-carbonsäure,

9(S)-[[Hydroxy(2-phenäthyl)phosphinyl]methyl]-octahydro-10-oxo-6H-pyridazo[1,2-a][1,2]diazepin-1(S)-carbonsäure und

9(S)-[Hydroxy(4-phenylbutyl)phosphinyl]-octahydro-10-oxo-6H-pyridazo[1,2-a][1,2]diazepin-1(S)-carbonsäure.

Weitere interessante Verbindungen der Formel I sind:

Methyl 9(S)-dimethoxyphosphinylmethyl-octahydro-6,10-dioxo-6H-pyridazo[1,2-a][1,2]diazepin-1(S)-carboxylat,

Methyl 9(S)-dimethoxyphosphinylmethyl-octahydro-10-oxo-6H-pyridazo[1,2-a][1,2]diazepin-1(S)-carboxylat,

Methyl octahydro-9(S)-phosphonomethyl-10-oxo-6H-pyridazo[1,2-a][1,2]diazepin-1(S)-carboxylat,

Methyl 9(S)-[[(äthoxy)methylphosphinyl]methyl]-octahydro-6,10-dioxo-6H-pyridazo[1,2-a][1,2]diazepin-1(S)-carboxylat,

Methyl 9(S)-diäthoxyphosphinyl-octahydro-6,10-dioxo-6H-pyridazo[1,2-a][1,2]diazepin-1(S)-carboxylat,

Methyl 9(S)-diäthoxyphosphinylmethyl-octahydro-6,10-dioxo-6H-pyridazo[1,2-a][1,2]diazepin-1(S)-carboxylat,

Methyl 9(S)-[[äthoxy(3-phenylpropyl)phosphinyl]-methyl]-octahydro-6,10-dioxo-6H-pyridazo[1,2-a][1,2]diazepine-1(S)-carboxylat,

Benzyl 9(S)-[[benzyloxy(phenäthyl)phosphinyl]amino]-octahydro-10-oxo-6H-pyridazo[1,2-a][1,2]diazepin-1(S)-carboxylat,

tert.Butyl 9(S)-(dibenzylphosphonoxy)-octahydro-6,10-dioxo-6H-pyridazo[1,2-a][1,2]diazepin-1(S)-carboxylat,

tert.Butyl 9(S)-[benzyloxy(phenäthyl)phosphinyloxy]-octahydro-6,10-dioxo-6H-pyridazo[1,2-a][1,2]diazepin-1(S)-carboxylat,

Benzyl 9(S)-[(dibenzyloxyphosphinyl)amino]-octahydro-10-oxo-6H-pyridazo[1,2-a][1,2]diazepin-1(S)-carboxylat und

9(S)-[Dibenzyloxyphosphinyl)amino]-octahydro-10-oxo-6H-pyridazo[1,2-a][1,2]diazepin-1(S)-carbonsäure.

Weitere Beispiele interessanter Verbindungen der Formel I sind:

tert.Butyl 9(S)-[benzyloxy(4-phenylbutyl)phosphinyloxy]-octahydro-6,10-dioxo-6H-pyridazo[1,2-a][1,2]diazepin-1(S)-carboxylat,

9(S)-[Hydroxy(4-phenylbutyl)phosphinyloxy]-octahydro-6,10-dioxo-6H-pyridazo[1,2-a][1,2]diazepin-1(S)-carbonsäure,

Benzyl 9(S)-[[benzyloxy(benzyl)phosphinyl]amino]-octahydro-10-oxo-6H-pyridazo[1,2-a][1,2]diazepin-1(S)-carboxylat,

Benzyl 9(S)-[[benzyloxy(3-phenylpropyl)phosphinyl]-amino]-octahydro-10-oxo-6H-pyridazo[1,2-a][1,2]diazepin-1(S)-carboxylat,

Benzyl 9(S)-[[benzyloxy(4-phenylbutyl)phosphinyl]-amino]-octahydro-10-oxo-6H-pyridazo[1,2-a][1,2]diazepin-1(S)-carboxylat,

9(S)-[[Hydroxy(benzyl)phosphinyl]amino]-octahydro-10-oxo-6H-pyridazo[1,2-a][1,2]diazepin-1(S)-carbonsäure,

9(S)-[[Hydroxy(3-phenylpropyl)phosphinyl]amino]-octahydro-10-oxo-6H-pyridazo[1,2-a][1,2]diazepin-1(S)-carbonsäure,

9(S)-[[Hydroxy(4-phenylbutyl)phosphinyl]amino]-octahydro-10-oxo-6H-pyridazo[1,2-a][1,2]diazepin-1(S)-carbonsäure,

Methyl 9(S)-[(äthoxy)(5-phenylpentyl)phosphinyl]-octahydro-6,10-dioxo-6H-pyridazo[1,2-a][1,2]diazepin-1(S)-carboxylat,

Methyl 9(S)-[(äthoxy)(4-phenylbutyl)phosphinyl]-octahydro-6,10-dioxo-6H-pyridazo[1,2-a][1,2]diazepin-1(S)-carboxylat,

Methyl 9(S)-[(äthoxy)(3-phenylpropyl)phosphinyl]-octahydro-6,10-dioxo-6H-pyridazo[1,2-a][1,2]diazepin-1(S)-carboxylat,

Methyl 9(S)-[(äthoxy)(phenäthyl)phosphinyl]-octahydro-6,10-dioxo-6H-pyridazo[1,2-a][1,2]diazepin-1(S)-car-

boxylat,

9(S)-[Hydroxy(5-phenylpentyl)phosphinyl]-octahydro-6,10-dioxo-6H-pyridazo[1,2-a][1,2]diazepin-1(S)-carbonsäure,

9(S)-[Hydroxy(4-phenylbutyl)phosphinyl]-octahydro-6,10-dioxo-6H-pyridazo[1,2-a][1,2]diazepin-1(S)-carbonsäure,

9(S)-[Hydroxy(3-phenylpropyl)phosphinyl]-octahydro-6,10-dioxo-6H-pyridazo[1,2-a][1,2]diazepin-1(S)-carbonsäure,

9(S)-[Hydroxy(phenäthyl)phosphinyl]octahydro-6,10-dioxo-6H-pyridazo[1,2-a][1,2]diazepin-1(S)-carbonsäure,

Methyl 9(S)-[[äthoxy(phenäthyl)phosphinyl]methyl]-octahydro-6,10-dioxo-6H-pyridazo[1,2-a][1,2]diazepin-1(S)-carboxylat,

9(S)-[[Hydroxy(phenäthyl)phosphinyl]methyl]octahydro-6,10-dioxo-6H-pyridazo[1,2-a][1,2]diazepin-1(S)-carbonsäure,

Methyl 9(S)-[[äthoxy(phenäthyl)phosphinyl]methyl]-octahydro-10-oxo-6H-pyridazo[1,2-a][1,2]diazepin-1(S)-carboxylat,

Methyl 9(S)-[(äthoxy)(4-phenylbutyl)phosphinyl]-octahydro-10-oxo-6H-pyridazo[1,2-a][1,2]diazepin-1(S)-carboxylat,

Aethyl 9(S)-[[(4-benzyloxybutyl)(äthoxy)phosphinyl]-methyl]-octahydro-6,10-dioxo-6H-pyridazo[1,2-a][1,2]diazepin-1(S)-carboxylat,

9(S)-[[(4-Benzyloxybutyl)(hydroxy)phosphinyl]methyl]-octahydro-6,10-dioxo-6H-pyridazo[1,2-a][1,2]diazepin-1(S)-carbonsäure,

9(S)-[[(4-Hydroxybutyl)(hydroxy)phosphinyl]methyl]-octahydro-6,10-dioxo-6H-pyridazo[1,2-a][1,2]diazepin-1(S)-carbonsäure,

9(S)-[[(4-Aminobutyl)(äthoxy)phosphinyl]methyl]-octahydro-6,10-dioxo-6H-pyridazo[1,2-a][1,2]diazepin-1(S)-carboxylat,

9(S)-[[(4-Aminobutyl)(hydroxy)phosphinyl]methyl]-octahydro-6,10-dioxo-6H-pyridazo[1,2-a][1,2]diazepin-1(S)-carbonsäure,

tert.Butyl octahydro-9(S)-[[methoxy(4-nitrophenäthyl)-phosphinyl]methyl]-6,10-dioxo-6H-pyridazo[1,2-a][1,2]diazepin-1(S)-carboxylat,

Octahydro-9(S)-[[methoxy(4-nitrophenäthyl)phosphinyl]-methyl]-6,10-dioxo-6H-pyridazo[1,2-a][1,2]diazepin-1(S)--carbonsäure,

Octahydro-9(S)-[[hydroxy(4-nitrophenäthyl)phosphinyl]-methyl]-6,10-dioxo-6H -pyridazo[1,2-a][1,2]diazepin-1(S)--carbonsäure,

9(S)-[[(4-Aminophenäthyl)hydroxyphosphinyl]methyl]-octahydro-6,10-dioxo-6H-pyridazo[1,2-a][1,2]diazepin-1(S)-carbonsäure,

9(S)-[[(4-Aminophenäthyl)methoxyphosphinyl]methyl]-octahydro-6,10-dioxo-6H-pyridazo[1,2-a][1,2]diazepin-1(S)-carbonsäure und

Octahydro 9(S)-[[(hydroxy)[4-(1-iminoäthylamino)phenäthyl]phosphinyl]methyl]-6,10-dioxo-6H-pyridazo[1,2-a][1,2]-diazepin-1(S)-carbonsäure.

Die Verbindungen der Formel I und deren pharmazeutisch verwendbare Salze können erfindungsgemäss dadurch hergestellt werden, dass man

a) zur Herstellung einer Verbindung der Formel I, worin $R^1$ Alkyl, Aralkyl, Alkoxyalkyl, Acylaminoalkyl, Dialkylaminoalkyl, Alkoxycarbonylaminoalkyl, Alkoxycarbonylalkyl, Alkoxy oder Aralkoxy, $R^2$ und $R^3$ je Alkyl oder Aralkyl, $R^4$ und $R^5$ zusammen eine Oxogruppe und X $-(CH_2)_n-$ bedeuten, worin n die oben angegebene Bedeutung besitzt, eine Verbindung der allgemeinen Formel

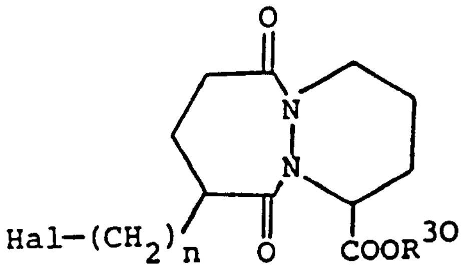

II

worin n die oben angegebenen Bedeutungen besitzt und $R^{30}$ Alkyl oder Aralkyl und Hal ein Halogenatom bedeuten,

mit einer Verbindung der allgemeinen Formel

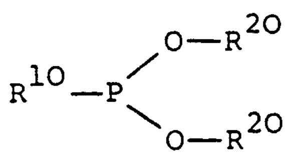

III

worin $R^{10}$ Alkyl, Aralkyl, Aralkoxyalkyl, Acylaminoalkyl, Dialkylaminoalkyl, Alkoxycarbonylaminoalkyl, Alkoxycarbonylalkyl, Alkoxy oder Aralkoxy und $R^{20}$ Alkyl oder Aralkyl bedeuten,

umsetzt,

oder

b) zur Herstellung einer Verbindung der Formel I, worin $R^1$ Alkyl, Aralkyl, Aralkoxyalkyl, Acylaminoalkyl, Dialkylaminoalkyl, Alkoxycarbonylaminoalkyl, Alkoxycarbonylalkyl, Alkoxy oder Aralkoxy, $R^2$ und $R^3$ je Alkyl oder Aralkyl und X ein Sauerstoffatom oder die Gruppe $-NR^6-$ bedeuten, worin $R^6$ Wasserstoff, Alkyl oder Aralkyl bedeutet, eine

Verbindung der allgemeinen Formel

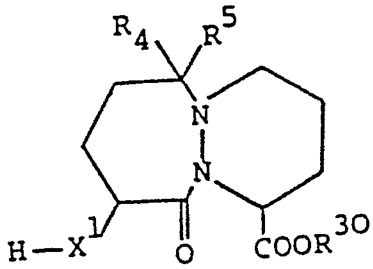

IV

worin $R^4$, $R^5$ und $R^{30}$ die oben angegebene Bedeutung besitzen und $X^1$ ein Sauerstoffatom oder die Gruppe $-NR^6-$ bedeutet, worin $R^6$ die oben angegebene Bedeutung besitzt,

mit einer Verbindung der allgemeinen Formel

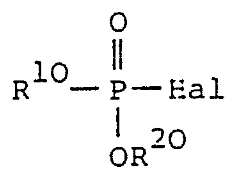

V

worin $R^{10}$, $R^{20}$ und Hal die oben angegebene Bedeutung besitzen,

umsetzt,

oder

c) zur Herstellung einer Verbindung der Formel I, worin $R^2$ Alkyl oder Aralkyl, $R^4$ und $R^5$ zusammen eine Oxogruppe und X $-CH_2-$ bedeuten, eine Verbindung der allgemeinen Formel

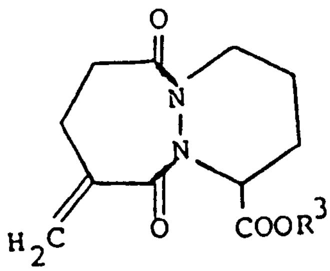

VI

worin $R^3$ die oben angegebene Bedeutung besitzt, mit einer Verbindung der allgemeinen Formel

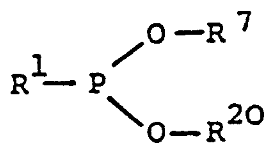

VII

worin $R^1$ und $R^{20}$ die oben angegebene Bedeutung besitzen und $R^7$ Alkyl, Aralkyl oder Trialkylsilyl bedeutet,

umsetzt,

oder

d) zur Herstellung einer Verbindung der Formel I, worin $R^1$ Alkyl, Aralkyl, Aralkoxyalkyl, Aminoalkyl, Acylaminoalkyl, Monoalkylaminoalkyl, Dialkylaminoalkyl, Alkoxycarbonylaminoalkyl, Alkoxycarbonylalkyl, Alkoxy oder Aralkoxy, $R^2$ und $R^3$ je Alkyl oder Aralkyl und $R^4$ und $R^5$ je Wasserstoff bedeuten, eine Verbindung der Formel I, worin $R^1$ Alkyl, Aralkyl, Aralkoxyalkyl, Aminoalkyl, Acylaminoalkyl, Monoalkylaminoalkyl, Dialkylaminoalkyl, Alkoxycarbonylaminoalkyl, Alkoxycarbonylalkyl, Alkoxy oder Aralkoxy,

$R^2$ und $R^3$ je Alkyl oder Aralkyl und $R^4$ und $R^5$ zusammen eine Oxogruppe bedeuten, reduziert, oder

e) zur Herstellung einer Verbindung der Formel I, worin $R^1$ Hydroxy, Alkoxy oder Aralkoxy, $R^2$ Wasserstoff, Alkyl oder Aralkyl und/oder $R^3$ Wasserstoff, Alkyl oder Aralkyl bedeuten, mit der Auflage, dass entweder $R^1$ Hydroxy und/oder mindestens einer von $R^2$ und $R^3$ Wasserstoff bedeutet, in einer Verbindung der Formel I, worin $R^1$ Hydroxy, Alkoxy oder Aralkoxy, $R^2$ Wasserstoff, Alkyl oder Aralkyl und/oder $R^3$ Wasserstoff, Alkyl oder Aralkyl bedeuten, mit der Massgabe, dass entweder $R^1$ von Hydroxy und/oder mindestens einer von $R^2$ und $R^3$ von Wasserstoff verschieden ist, die Estergruppe abspaltet, oder

f) zur Herstellung einer Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy oder Aralkoxy, $R^2$ und $R^3$ je Alkyl oder Aralkyl und X die Gruppe $-NR^{61}-$ bedeuten, worin $R^{61}$ Alkyl oder Aralkyl bedeutet, eine Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy oder Aralkoxy, $R^2$ und $R^3$ je Alkyl oder Aralkyl und X die Gruppe -NH- bedeuten, alkyliert oder aralkyliert, oder

g) zur Herstellung einer Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkyl oder Aralkyl, $R^2$ Alkyl oder Aralkyl und $R^3$ Alkyl oder Aralkyl bedeuten, eine Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkyl oder Aralkyl, $R^2$ Alkyl oder Aralkyl und $R^3$ Wasserstoff bedeuten, verestert, oder

h) zur Herstellung einer Verbindung der Formel I, worin $R^1$ Hydroxyalkyl und $R^2$ und $R^3$ je Wasserstoff oder Alkyl bedeuten, eine Verbindung der Formel I, worin $R^1$ Aryloxyalkyl und $R^2$ und $R^3$ je Wasserstoff oder Alkyl bedeuten, katalytisch hydriert, oder

i) zur Herstellung einer Verbindung der Formel I, worin $R^1$ Haloalkyl und $R^2$ und $R^3$ je Alkyl oder Aralkyl bedeuten, eine Verbindung der Formel I, worin $R^1$ Hydroxyalkyl und $R^2$ und $R^3$ je Alkyl oder Aralkyl bedeuten, halogeniert, oder

j) zur Herstellung einer Verbindung der Formel I, worin $R^1$ Aminoalkyl oder Monoalkylaminoalkyl und $R^2$ und $R^3$ je Alkyl oder Aralkyl bedeuten, die Hydroxyalkylgruppe in einer Verbindung der Formel I, worin $R^1$ Hydroxyalkyl und $R^2$ und $R^3$ je Alkyl oder Aralkyl bedeuten, in eine Aminoalkyl- oder Monoalkylaminoalkylgruppe überführt, oder

k) zur Herstellung einer Verbindung der Formel I, worin $R^1$ Aminoalkyl oder Monoalkylaminoalkyl und $R^2$ und $R^3$ je Alkyl oder Aralkyl bedeuten, eine Verbindung der Formel I, worin $R^1$ Haloalkyl und $R^2$ und $R^3$ je Alkyl oder Aralkyl bedeuten, mit Ammoniak oder einem Monoalkylamin umsetzt, oder

l) zur Herstellung einer Verbindung der Formel I, worin $R^1$ Acylaminoalkyl und $R^2$ und $R^3$ je Alkyl oder Aralkyl bedeuten, eine Verbindung der Formel I, worin $R^1$ Aminoalkyl und $R^2$ und $R^3$ je Alkyl oder Aralkyl bedeuten, acyliert, oder

m) zur Herstellung einer Verbindung der Formel I, worin $R^1$ Carboxyalkyl und $R^2$ und $R^3$ je Alkyl oder Aralkyl bedeuten, eine Verbindung der Formel I, worin $R^1$ Hydroxyalkyl und $R^2$ und $R^3$ je Alkyl oder Aralkyl bedeuten, oxydiert, und

n) erwünschtenfalls eine im Substituenten $R^1$ in einer Verbindung der Formel I vorhandene nitrosubstituierte Phenylgruppe zur aminosubstituierten Phenylgruppe reduziert, und/oder

o) erwünschtenfalls eine im Substituenten $R^1$ in einer Verbindung der Formel I vorhandene aminosubstituierte Phenylgruppe durch Umsetzen mit einem Alkylcyanid in Gegenwart eines Trialkylsilylhalogenids in eine Iminoalkylaminogruppe überführt, und/oder

p) erwünschtenfalls ein erhaltenes Gemisch von diastereoisomeren Racematen in die entsprechenden diastereoisomeren Racemate oder optisch reinen Diastereoisomeren auftrennt, und/oder

q) erwünschtenfalls ein erhaltenes Racemat in die beiden optischen Antipoden auftrennt, und

r) erwünschtenfalls eine erhaltene Verbindung der Formel I in ein pharmazeutisch verwendbares Salz überführt.

Die Umsetzung einer Verbindung der Formel II mit einer Verbindung der Formel III gemäss Verfahrensvariante a) kann in bekannter Weise in Gegenwart oder Abwesenheit eines inerten organischen Lösungsmittels durchgeführt werden. Falls ein inertes organisches Lösungsmittel verwendet wird, so kommt zweckmässigerweise ein Kohlenwasserstoff, wie Benzol, Toluol und dergleichen, ein halogenierter Kohlenwasserstoff, wie Methylenchlorid, Chlorbenzol und dergleichen, ein Amid, wie Dimethylformamid, und dergleichen in Frage.

Die Umsetzung erfolgt zweckmässig bei erhöhter Temperatur, vorzugsweise bei der Rückflusstemperatur des Reaktionsgemisches. Unter gewissen Umständen kann es vorteilhaft sein, die Umsetzung in einer Inertgasatmosphäre, z.B. Stickstoff, Argon und dergleichen, durchzuführen. Das Halogenatom in der Verbindung der Formel II ist vorzugsweise ein Bromatom.

Die Umsetzung einer Verbindung der Formel IV mit einer Verbindung der Formel V gemäss Verfahrensvariante b) kann in bekannter Weise in Gegenwart einer Base und eines inerten organischen Lösungsmittels bei ungefähr Raumtemperatur durchgeführt werden. Wenn beispielsweise eine Verbindung der Formel IV, worin $X^1$ ein Sauerstoffatom bedeutet, verwendet wird, wird die Reaktion zweckmässigerweise so durchgeführt, dass man zunächst diese Verbindung mit einem Alkalimetallhydrid, wie Natriumhydrid, in einem inerten organischen Lösungsmittel, wie einem cyclischen Aether, z.B. Tetrahydrofuran, umsetzt und danach die zweckmässigerweise im gleichen organischen Lösungsmittel gelöste Verbindung der Formel V zugibt. Wenn - wiederum beispielsweise - eine Verbindung der Formel IV, worin $X^1$ die Gruppe $-NR^6-$ bedeutet, verwendet wird, kann die Umsetzung zweckmässigerweise in Gegenwart eines tertiären Amins, wie eines Tri(niederalkyl)amins, z.B. Triäthylamin, Diisopropyläthylamin und dergleichen, und in einem inerten organischen Lösungsmittel, wie einem halogenierten Kohlenwasserstoff, z.B. Methylenchlorid, Chloroform, Tetrachlorkohlenstoff und dergleichen, durchgeführt werden.

Die Umsetzung einer Verbindung der Formel VI mit einer Verbindung der Formel VII gemäss Verfahrensvariante c) kann in Gegenwart oder Abwesenheit eines inerten organischen Lösungsmittels durchgeführt werden. Falls ein Lösungsmittel verwendet wird, so kommt zweckmässigerweise ein halogenierter Kohlenwasserstoff, z.B. Methylenchlorid, Chloroform, Chlorbenzol und dergleichen, oder ein aromatischer Kohlenwasserstoff, z.B. Benzol, Toluol, ein Xylol und dergleichen, in Frage. Die Umsetzung wird zweckmässigerweise bei einer

Temperatur zwischen ungefähr 20°C und ungefähr 150°C durchgeführt. Vorteilhafterweise wird die Reaktion unter einer Inertgasatmosphäre, wie Stickstoff, Argon und dergleichen, durchgeführt. Falls eine Verbindung der Formel VII, worin $R^7$ Trialkylsilyl, z.B. Trimethylsilyl, bedeutet verwendet wird, wird diese Verbindung zweckmässigerweise in situ hergestellt. Wenn eine Verbindung der Formel VI, worin $R^3$ Wasserstoff bedeutet, mit einer Verbindung der Formel VII, worin $R^7$ Alkyl oder Aralkyl bedeutet, umgesetzt wird, erhält man unter gewissen Umständen in Abhängigkeit von den verwendeten Reaktionsbedingungen eine Verbindung der Formel I, worin $R^3$ Alkyl oder Aralkyl bedeutet.

Die Reduktion einer Verbindung der Formel I, worin $R^1$ Alkyl, Aralkyl, Aralkoxyalkyl, Aminoalkyl, Acylaminoalkyl, Monoalkylaminoalkyl, Dialkylaminoalkyl, Alkoxycarbonylaminoalkyl, Alkoxycarbonylalkyl, Alkoxy oder Aralkoxy, $R^2$ Alkyl oder Aralkyl, $R^3$ Alkyl oder Aralkyl und $R^4$ und $R^5$ zusammen eine Oxogruppe bedeuten, gemäss Verfahrensvariante d) erfolgt zweckmässig unter Verwendung eines Borankomplexes, wie Boran/Tetrahydrofuran, Boran/Dimethylsulfid, Boran/N,N-Diäthylanilin oder eines ähnlichen Komplexes. Diese Reduktion wird vorteilhafterweise in einem inerten organischen Lösungsmittel bei Raumtemperatur oder einer Temperatur unterhalb der Raumtemperatur durchgeführt, beispielsweise unter Verwendung eines Boran/Tetrahydrofuran--Komplexes in Tetrahydrofuran bei ungefähr 0°C bis 20°C.

Die Abspaltung der Estergruppe in einer Verbindung der Formel I, worin $R^1$ Alkoxy oder Aralkoxy, $R^2$ Alkyl oder Aralkyl und/oder $R^3$ Alkyl oder Aralkyl bedeuten, gemäss Verfahrensvariante e) erfolgt nach an sich bekannten Methoden. Beispielsweise kann eine Verbindung der Formel I, worin $R^1$ Alkoxy oder Aralkoxy und $R^2$ Alkyl oder Aralkyl bedeuten, in eine entsprechende Verbindung der Formel I übergeführt werden, worin $R^1$ Hydroxy und $R^2$ Wasserstoff bedeuten, indem man die erstgenannte Verbindung zweckmässigerweise bei ungefähr Raumtemperatur mit Trimethylsilylbromid umsetzt. Wiederum beispielsweise kann eine Verbindung der Formel I, worin $R^1$ Alkoxy oder Aralkoxy und $R^2$ Alkyl oder Aralkyl bedeuten, durch Umsetzen mit Lithiumthiocyanat, zweckmässigerweise in einem inerten organischen Lösungsmittel, wie einem aliphatischen Keton, z.B. Aceton, 2-Butanon und dergleichen, und bei erhöhter Temperatur, beispielsweise der Rückflusstemperatur des Reaktionsgemisches, in die entsprechende Verbindung der Formel I übergeführt werden, worin $R^1$ Alkoxy oder Aralkoxy und $R^2$ Wasserstoff bedeuten. Die Ueberführung einer Verbindung der Formel I, worin $R^3$ Alkyl oder Aralkyl bedeutet, in die entsprechende Verbindung der Formel I, worin $R^3$ Wasserstoff bedeutet, kann beispielsweise durch Umsetzen mit einer Base oder, falls die Alkylgruppe die tert.Butylgruppe ist, durch Umsetzen mit einer Säure bewerkstelligt werden. Geeignete Basen sind Alkalimetallhydroxide, z.B. Natriumhydroxid, Kaliumhydroxid und dergleichen, und Ammoniumhydroxid. Die Umsetzung kann bei einer Temperatur zwischen ungefähr Raumtemperatur und dem Siedepunkt des Reaktionsgemisches, vorzugsweise bei ungefähr Raumtemperatur, durchgeführt werden. Wasserfreie Trifluoressigsäure ist eine besonders geeignete Säure zur Abspaltung der Estergruppe in einer Verbindung der Formel I, worin $R^3$ tert.Butyl bedeutet, wobei die Umsetzung in diesem Falle vorzugsweise bei ungefähr Raumtemperatur erfolgt. Eine Verbindung der Formel I, worin $R^3$ Aralkyl bedeutet, kann auch durch Hydrogenolyse in an sich bekannter Weise in eine entsprechende Verbindung der Formel I übergeführt werden, worin $R^3$ Wasserstoff bedeutet.

Die Alkylierung oder Aralkylierung einer Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy oder Aralkoxy, $R^2$ Alkyl oder Aralkyl, $R^3$ Alkyl oder Aralkyl und X die Gruppe -NH- bedeuten, gemäss Verfahrensvariante f) kann nach

an sich bekannten Methoden durchgeführt werden. Beispielsweise kann eine Verbindung der Formel I mit einem geeigneten Alkylhalogenid, z.B. Methylbromid, Aethylchlorid und dergleichen, oder einem geeigneten Aralkylhalogenid, z.B. Benzylbromid oder dergleichen, in Gegenwart einer Base umgesetzt werden.

Die Veresterung gemäss Verfahrensvariante g) kann nach an sich bekannten Methoden durchgeführt werden. Beispielsweise kann die Veresterung durch Umsetzen mit einem geeigneten Alkohol, z B. Methanol, Aethanol, Benzylalkohol und dergleichen, in Gegenwart einer Mineralsäure, z.B. Chlorwasserstoffsäure und dergleichen, oder mit einem geeigneten Diazoalkan, z.B. Diazomethan, Phenyldiazomethan und dergleichen, durchgeführt werden. Ein weiteres Verfahren zur Herstellung eines tert.Butylesters umfasst die Veresterung unter Verwendung von Isobuten in Gegenwart von Schwefelsäure.

Die katalytische Hydrierung gemäss Verfahrensvariante h) kann nach an sich bekannten Methoden durchgeführt werden, beispielsweise in Gegenwart eines Edelmetallkatalysators, wie eines Palladiumkatalysators, in einem inerten organischen Lösungsmittel, z.B. einem Alkanol, wie Aethanol und dergleichen, zweckmässigerweise bei ungefähr Raumtemperatur und Atmosphärendruck.

Uebliche Methoden können zur Halogenierung gemäss Verfahrensvariante i) verwendet werden. Beispielsweise kann die Halogenierung durch Umsetzen mit einem geeigneten Halogenierungsmittel, wie einem Thionylhalogenid, z.B. Thionylchlorid etc., einem Phosphorhalogenid, z.B. Phosphortrichlorid, Phosphorpentachlorid etc., oder dergleichen, durchgeführt werden. Alternativ dazu kann man die Halogenierung auch so durchführen, dass man zunächst die Verbindung der Formel I, worin $R^1$ Hydroxyalkyl und $R^2$ und $R^3$ je Alkyl oder Aryl bedeuten, in das entsprechende Alkylsulfonat, z.B. Mesylat, oder Arylsulfonat, z.B. Tosylat, überführt und dieses danach

mit einem Alkalimetallhalogenid, z.B. Natriumfluorid, Natriumchlorid, Lithiumchlorid und dergleichen, in Aceton oder mit einem Pyridinhydrohalogenid bei einer Temperatur zwischen ungefähr 0°C und 100°C umsetzt.

Die Ueberführung der Hydroxygruppe in einer Verbindung der Formel I, worin $R^1$ Hydroxyalkyl und $R^2$ und $R^3$ je Alkyl oder Aralkyl bedeuten, in eine Aminoalkyl- oder Monoalkylaminoalkylgruppe gemäss Verfahrensvariante j) kann nach an sich bekannten Methoden durchgeführt werden. Beispielsweise kann eine solche Verbindung zunächst in das entsprechende Alkylsulfonat, z.B. Mesylat, oder Arylsulfonat, z.B. Tosylat, übergeführt werden, welches danach mit Ammoniak oder einem geeigneten Monoalkylamin, z.B. Methylamin, Aethylamin etc., umgesetzt wird. Gemäss einer alternativen Ausführungsform kann das Alkylsulfonat oder Arylsulfonat mit einem Alkalimetallazid, z.B. Natriumazid, in bekannter Weise, beispielsweise einem inerten organischen Lösungsmittel, wie 2-Butanon, und bei erhöhter Temperatur, z.B. bei der Rückflusstemperatur des Reaktionsgemisches, unter Bildung der entsprechenden Azidoalkylverbindung umgesetzt werden, welche danach durch katalytische Hydrierung in an sich bekannter Weise in die erwünschte Aminoalkylverbindung übergeführt werden kann.

Die Umsetzung einer Verbindung der Formel I, worin $R^1$ Haloalkyl und $R^2$ und $R^3$ je Alkyl oder Aralkyl bedeuten, mit Ammoniak oder einem Monoalkylamin, z.B. Methylamin, Aethylamin und dergleichen, gemäss Verfahrensvariante k) kann nach konventionellen Methoden durchgeführt werden, beispielsweise in einem inerten organischen Lösungsmittel und in Gegenwart eines geeigneten säurebindenden Mittels.

Konventionelle Methoden können für die Acylierung einer Verbindung der Formel I, worin $R^1$ Aminoalkyl und $R^2$ und $R^3$ je Alkyl oder Aralkyl bedeuten, gemäss Verfahrensvariante l) verwendet werden. Beispielsweise kann die Acylierung unter Verwendung eines geeigneten Acylhalogenides, z.B. eines Acylchlorides, wie Acetylchlorid, in Gegenwart eines geeigneten Säurebindemittels durchgeführt werden.

Die Oxidation gemäss Verfahrensvariante m) kann ebenfalls nach bekannten Methoden durchgeführt werden, beispielsweise durch Umsetzen mit einem Oxidationsmittel, wie einem Alkalimetalldichromat, z.B. Kaliumdichromat und dergleichen.

Die Reduktion einer nitrosubstituierten Phenylgruppe zu einer aminosubstituierten Phenylgruppe gemäss Verfahrensvariante n) erfolgt nach an sich bekannten Methoden, beispielsweise durch katalytische Hydrierung in Gegenwart eines Edelmetallkatalysators, wie eines Palladiumkatalysators, z.B. Palladium auf Kohle, in einem inerten organischen Lösungsmittel, z.B. einem Alkanol, wie Methanol, Aethanol und dergleichen.

Die Ueberführung einer aminosubstituierten Phenylgruppe in eine Iminoalkylaminogruppe gemäss Verfahrensvariante o) kann unter Verwendung von überschüssigem Alkylcyanid, z.B. Acetonitril, Propionitril und dergleichen, durchgeführt werden, wobei dieses auch als Lösungsmittel dient. Das bevorzugte Trialkylsilylhalogenid ist das Trimethylsilylbromid, obwohl auch Trialkylsilylhalogenide, wie Trimethylsilylchlorid und dergleichen, verwendet werden kann. Die Umsetzung erfolgt zweckmässigerweise bei ungefähr Raumtemperatur.

Die Auftrennung von diastereoisomeren Mischungen in die diastereoisomeren Racemate oder optisch reinen Diastereoisomeren gemäss Verfahrensvariante p) erfolgt nach an sich bekannten Methoden, beispielsweise durch Chromatographie, z.B. an Silicagel, unter Verwendung eines geeigneten Lösungsmittelsystems, z.B. Aethylacetat/n-Hexan.

Die Auftrennung eines Racemates in die beiden optischen Antipoden gemäss Verfahrensvariante q) kann in bekannter Weise durchgeführt werden, beispielsweise durch Umsetzen mit einer geeigneten optisch aktiven Säure oder einer geeigneten optisch aktiven Base in Abhängigkeit von der Natur des zu spaltenden Racemates, Abtrennen des erhaltenen optisch aktiven Salzes, z.B. durch fraktionierte Kristallisation, und nötigenfalls Freisetzen der optisch einheitlichen Verbindungen aus diesen Salzen nach bekannten Methoden.

Die Ueberführung einer Verbindung der Formel I in ein pharmazeutisch verwendbares Salz gemäss Verfahrensvariante r) kann in bekannter Weise erfolgen. Beispielsweise kann eine Verbindung der Formel I, welche sauer ist, durch Umsetzen mit einer geeigneten Base, wie einem Alkalimetallhydroxid, z.B. Natriumhydroxid, Kaliumhydroxid und dergleichen, einem Erdalkalimetallhydroxid, z.B. Calciumhydroxid, Magnesiumhydroxid und dergleichen, Ammoniumhydroxid oder einem organischen Amin, z.B. Dicyclohexylamin und dergleichen, in ein pharmazeutisch verwendbares Salz übergeführt werden. Eine basische Verbindung der Formel I kann - ebenfalls wiederum beispielsweise - durch Umsetzen mit einer Säure, wie einer Mineralsäure, z.B. einer Halogenwasserstoffsäure, wie Bromwasserstoffsäure, Chlorwasserstoffsäure und dergleichen, Schwefelsäure, Phosphorsäure, Salpetersäure und dergleichen, oder einer organischen Säure, wie Essigsäure, Maleinsäure, Fumarsäure, Weinsäure, Zitronensäure, Salicylsäure, Methansulfonsäure, p-Toluolsulfonsäure und dergleichen, in ein pharmazeutisch verwendbares Salz übergeführt werden.

Die in Verfahrensvariante a) als Ausgangsstoffe eingesetzten Verbindungen der Formel II, worin n 0 bedeutet, sind bekannt.

Die ebenfalls in Verfahrensvariante a) als Ausgangsstoffe eingesetzten Verbindungen der Formel II, worin n 1

oder 2 bedeutet, sind neu und können hergestellt werden, indem man beispielsweise eine Verbindung der allgemeinen Formel

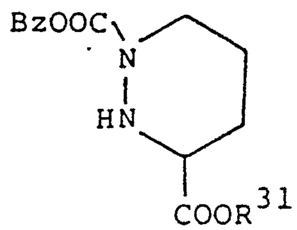

VIII

worin $R^{31}$ Alkyl und Bz Benzyl bedeuten, mit einer Verbindung der allgemeinen Formel

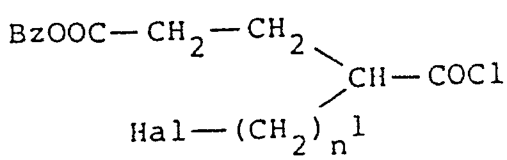

IX

worin Bz und Hal die oben angegebene Bedeutung besitzen und $n^1$ 1 oder 2 bedeutet,
umsetzt, die Benzyl- und Benzyloxycarbonylgruppen aus der erhaltenen Verbindung der allgemeinen Formel

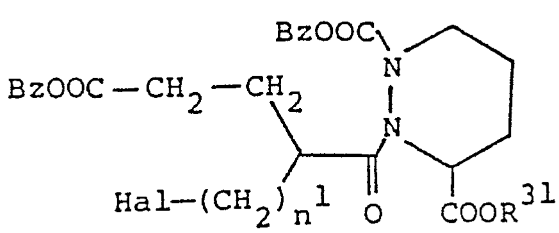

X

worin $R^{31}$, Bz, Hal und $n^1$ die oben angegebene Bedeutung besitzen,

abspaltet und die erhaltene Säure der allgemeinen Formel

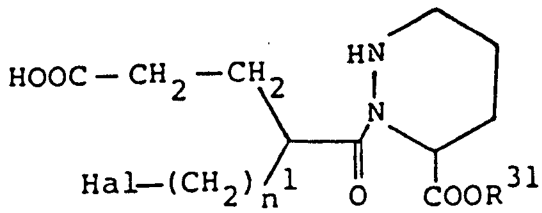

XI

worin $R^{31}$, Hal und $n^1$ die oben angegebene Bedeutung besitzen,

cyclisiert und, falls eine Verbindung der Formel II, worin n 1 oder 2 und $R^{30}$ Aralkyl bedeuten, erwünscht ist, die Estergruppe in der erhaltenen Verbindung der Formel II, worin n 1 oder 2 und $R^{30}$ Alkyl bedeuten, abspaltet und die erhaltene Säure zum entsprechenden Aralkylester verestert.

Die Umsetzung einer Verbindung der Formel VIII mit einer Verbindung der Formel IX kann in bekannter Weise erfolgen, beispielsweise in einem inerten organischen Lösungsmittel, z.B. einem halogenierten Kohlenwasserstoff, wie Methylenchlorid, und in Gegenwart einer Base, z.B. eines Alkalimetallcarbonats, wie Natriumcarbonat, oder eines Alkalimetallbicarbonats, wie Natriumbicarbonat, zweckmässigerweise bei ungefähr Raumtemperatur.

Die Abspaltung der Benzyl- und Benzyloxycarbonylgruppen aus einer Verbindung der Formel X kann nach allgemein bekannten Methoden durchgeführt werden, beispielsweise unter Verwendung von Wasserstoff in Gegenwart eines Edelmetallkatalysators, z.B. Palladium auf Kohle, oder, falls $R^{30}$ etwas anderes als tert.Butyl bedeutet, unter Verwendung von

Bromwasserstoff in Eisessig.

Die Cyclisation einer Säure der Formel XI kann ebenfalls in bekannter Weise durchgeführt werden. In einer bevorzugten Ausführungsform erfolgt die Cyclisation dadurch, dass man eine Säure der Formel XI in bekannter Weise mit einem geeigneten Halogenierungsmittel, wie einem Phosphorpentahalogenid, z.B. Phosphorpentachlorid und dergleichen, oder einem Thionylhalogenid, z.B. Thionylchlorid und dergleichen, in das entsprechende Säurehalogenid, z.B. das Säurechlorid, überführt, welches dann spontan zu Verbindung der Formel II cyclisiert, worin n 1 oder 2 und $R^{30}$ Alkyl bedeuten.

Die Abspaltung der Estergruppe aus einer Verbindung der Formel II, worin n 1 oder 2 und $R^{30}$ Alkyl bedeuten, unter Bildung einer entsprechenden Säure, d.h. einer der Formel II entsprechenden Verbindung, worin $R^{30}$ jedoch Wasserstoff bedeutet, kann beispielsweise durch Basenbehandlung oder, falls die Alkylgruppe die tert.Butylgruppe ist, durch Säurebehandlung bewerkstelligt werden. Geeignete Basen, die zu diesem Zwecke verwendet werden können, sind Alkalimetallhydroxide, z.B. Natriumhydroxid, Kaliumhydroxid und dergleichen, und Ammoniumhydroxid. Die Umsetzung kann bei einer Temperatur zwischen ungefähr Raumtemperatur und dem Siedepunkt des Reaktionsgemisches durchgeführt werden, vorteilhafterweise bei ungefähr Raumtemperatur. Wasserfreie Trifluoressigsäure ist eine speziell bevorzugte Säure für die Abspaltung der Estergruppe in einer Verbindung der Formel II, worin $R^{30}$ tert.Butyl bedeutet; in diesem Falle erfolgt die Umsetzung mit der Säure zweckmässigerweise bei ungefähr Raumtemperatur.

Die Veresterung der so erhaltenen Säure erfolgt in bekannter Weise, beispielsweise durch Umsetzen mit einem geeigneten Alkohol, z.B. Benzylalkohol und dergleichen, in Gegenwart einer Mineralsäure, z.B. Chlorwasserstoffsäure und dergleichen, oder mit einem geeigneten Diazoalkan, z.B.

Phenyldiazomethan und dergleichen.

Die Verbindungen der Formel II, worin n 1 bedeutet, können beispielsweise auch hergestellt werden, indem man die Benzyloxycarbonylgruppe aus einer Verbindung der obigen Formel VIII abspaltet, die erhaltene Verbindung der allgemeinen Formel

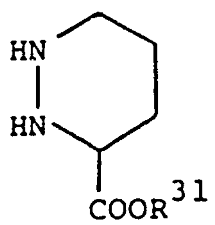

XII

worin $R^{31}$ die obigen angegebenen Bedeutungen besitzt, mit α-Methylenglutarsäureanhydrid umsetzt und die erhaltene Verbindung der allgemeinen Formel

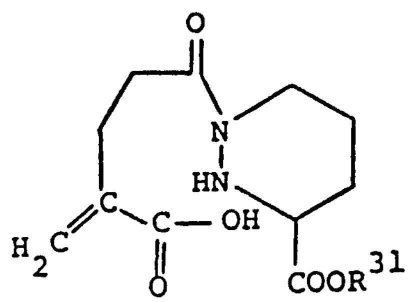

XIII

worin $R^{31}$ die oben angegebene Bedeutung besitzt, cyclisiert, die tert.Butylgruppe $R^{31}$ durch eine andere Alkylgruppe in der erhaltenen Verbindung der allgemeinen Formel

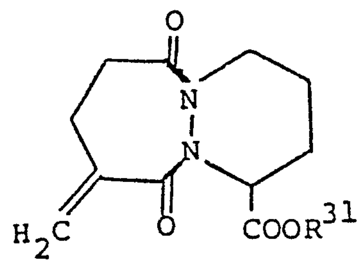

VIa

worin $R^{31}$ die oben angegebene Bedeutung besitzt, ersetzt, die Verbindung der Formel VIa, worin $R^{31}$ eine andere Alkylgruppe als die tert.Butylgruppe bedeutet, mit Halogenwasserstoff umsetzt und, falls eine Verbindung der Formel II, worin $R^{30}$ tert.Butyl oder Aralkyl bedeutet, erwünscht ist, die Estergruppe in der erhaltenen Verbindung der Formel II, worin n 1 und $R^{30}$ eine andere Alkylgruppe als die tert.Butylgruppe bedeuten, die Estergruppe abspaltet und die erhaltene Säure zum entsprechenden tert.Butyl- oder Aralkylester verestert.

Das Abspalten der Benzyloxycarbonylgruppe aus einer Verbindung der Formel VIII kann in bekannter Weise durchgeführt werden, beispielsweise mittels Wasserstoff in Gegenwart eines Katalysators, wie eines Edelmetallkatalysators, z.B. Palladium auf Kohle, und in Gegenwart eines inerten organischen Lösungsmittels, z.B. eines Alkanols, wie Methanol und dergleichen.

Umsetzung einer so erhaltenen Verbindung der Formel XII mit α-Methylglutarsäureanhydrid unter Bildung einer Verbindung der Formel XIII wird zweckmässigerweise in einem inerten organischen Lösungsmittel, wie einem halogenierten Kohlenwasserstoff, z.B. Methylenchlorid und dergleichen, oder einem Aether, z.B. Dioxan, Tetrahydrofuran und dergleichen, bei einer Temperatur zwischen ungefähr Raumtemperatur und der Rückflusstemperatur des Reaktionsgemisches,

vorzugsweise bei ungefähr Raumtemperatur, durchgeführt.

Die Cyclisation einer Verbindung der Formel XIII erfolgt in bekannter Weise. Gemäss einer bevorzugten Verfahrensführung wird die Cyclisation so durchgeführt, dass man eine Verbindung der Formel XIII in bekannter Weise mit einem geeigneten Halogenierungsmittel, wie einem Phosphorpentahalogenid, z.B. Phosphorpentachlorid und dergleichen, oder einem Thionylhalogenid, z.B. Thionylchlorid und dergleichen, in das entsprechende Säurehalogenid, z.B. Säurechlorid, überführt, welches dann spontan zur Verbindung der Formel VIa cyclisiert.

Wenn $R^{31}$ im Cyclisationsprodukt der Formel VIa tert.Butyl bedeutet, so kann diese Gruppe vor der Behandlung mit einem Halogenwasserstoff durch eine andere Alkylgruppe ersetzt werden. Dies erfolgt durch Behandeln mit wasserfreier Trifluoressigsäure unter Bildung der freien Säure, welche dann zu einem von tert.Butyl verschiedenen Alkylester verestert wird.

Eine so erhaltene Verbindung, d.h. eine Verbindung der Formel VIa, worin $R^{31}$ von tert.Butyl verschieden ist, wird dann mit einem Halogenwasserstoff zu einer Verbindung der Formel II, worin n 1 bedeutet und $R^{30}$ von tert.Butyl verschieden ist, umgesetzt. Bromwasserstoff ist dabei der bevorzugte Halogenwasserstoff. Vorzugsweise wird die Umsetzung in einem inerten organischen Lösungsmittel, wie einem halogenierten Kohlenwasserstoff, z.B. Methylenchlorid und dergleichen, bei ungefähr Raumtemperatur, durchgeführt.

Die Abspaltung der Estergruppe aus einer Verbindung der Formel II, worin n 1 bedeutet und $R^{30}$ von tert.Butyl verschieden ist, erfolgt in bekannter Weise durch Basenbehandlung. Geeignete Basen sind Alkalimetallhydroxide, z.B. Natriumhydroxid, Kaliumhydroxid und dergleichen, und Ammoniumhydroxid. Zweckmässigerweise wird die Umsetzung bei

einer Temperatur zwischen ungefähr Raumtemperatur und dem Siedepunkt des Reaktionsgemisches, vorzugsweise bei ungefähr Raumtemperatur, durchgeführt.

Die Ueberführung der erhaltenen Säure in einen tert.Butylester kann in bekannter Weise durchgeführt werden, beispielsweise durch Umsetzen mit Isobuten in Gegenwart von Schwefelsäure. Die Veresterung einer erhaltenen Säure zu einem Aralkylester kann in analoger Weise zu der weiter oben beschriebenen Methode durchgeführt werden.

Die Verbindungen der Formel II, worin n 1 oder 2 bedeutet, sind ebenfalls Gegenstand der vorliegenden Erfindung.

Die in Verfahrensvariante a) als Ausgangsstoffe eingesetzten Verbindungen der Formel III sind bekannt.

Die in Verfahrensvariante b) als Ausgangsstoffe eingesetzten Verbindungen der Formel IV, worin $X^1$ ein Sauerstoffatom bedeutet, sind neu und ebenfalls Gegenstand der vorliegenden Erfindung. Die Verbindungen der Formel IV, worin $X^1$ ein Sauerstoffatom und $R^{30}$ Alkyl bedeuten, können beispielsweise hergestellt werden, indem man eine Verbindung der Formel VIII mit einer Verbindung der allgemeinen Formel

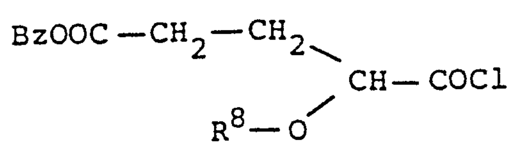

XV

worin Bz die oben angegebene Bedeutung besitzt und $R^8$ eine Alkanoylgruppe (z.B. Acetyl) bedeutet,

umsetzt, die Benzyl- und Benzyloxycarbonylgruppen aus der erhaltenen Verbindung der allgemeinen Formel

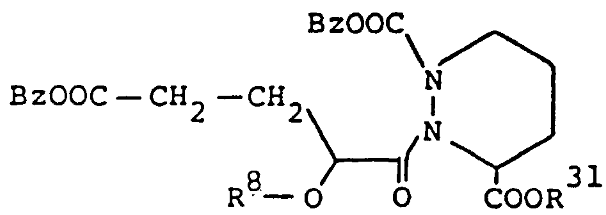

XVI

worin $R^8$, $R^{31}$ und Bz die oben angegebene Bedeutung besitzen,

abspaltet, die erhaltene Säure der allgemeinen Formel

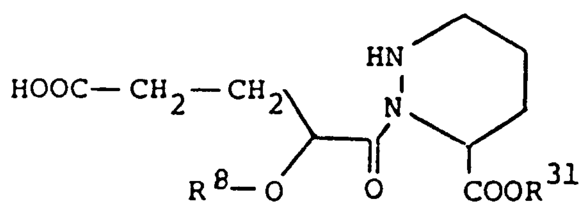

XVII

worin $R^8$ und $R^{31}$ die oben angegebene Bedeutung besitzen,

cyclisiert und die Alkanoylgruppe aus den so erhaltenen Verbindungen der allgemeinen Formel

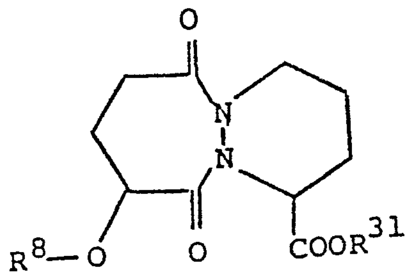

XVIII

worin $R^8$ und $R^{31}$ die oben angegebene Bedeutung besitzen,

abspaltet oder, falls eine Verbindung der Formel IV, worin $X^1$ ein Sauerstoffatom und $R^4$ und $R^5$ je Wasserstoff bedeuten, erwünscht ist, die erhaltene Verbindung der Formel XVIII reduziert und erst danach die Alkanoylgruppe abspaltet.

Die Umsetzung einer Verbindung der Formel VIII mit einer Verbindung der Formel XV kann in bekannter Weise erfolgen, beispielsweise in einem inerten organischen Lösungsmittel, z.B. einem Kohlenwasserstoff, wie Toluol, und in Gegenwart einer Base, z.B. eines Alkalimetallcarbonates, wie Natriumcarbonat, oder eines Alkalimetallbicarbonates, wie Natriumbicarbonat, zweckmässigerweise bei ungefähr Raumtemperatur.

Die Abspaltung der Benzyl- und Benzyloxycarbonylgruppen aus einer Verbindung der Formel XVI kann in bekannter Weise durchgeführt werden, beispielsweise mittels Wasserstoff in Gegenwart eines Edelmetallkatalysators, z.B. Palladium auf Kohle.

Die Cyclisation einer Säure der Formel XVII kann in bekannter Weise durchgeführt werden. Nach einer bevorzugten Ausführungsform wird die Cyclisation so durchgeführt, dass man eine Säure der Formel XVII in bekannter Weise mit einem geeigneten Halogenierungsmittel, wie einem Phosphorpentahalogenid, z.B. Phosphorpentachlorid und dergleichen, oder einem Thionylhalogenid, z.B. Thionylchlorid und dergleichen, zum entsprechenden Säurehalogenid, z.B. Säurechlorid, umsetzt, welches dann spontan zur Verbindung der Formel XVIII cyclisiert.

Die Abspaltung der Alkanoylgruppe aus einer Verbindung der Formel XVIII kann ebenfalls in bekannter Weise durchgeführt werden, beispielsweise durch Umsetzen mit einer geeigneten Base, wie einem Alkalimetallhydroxid, z.B. Natriumhydroxid und dergleichen, in einem inerten organischen Lösungsmittel, z.B. einem Alkanol, wie Methanol, Aethanol und dergleichen, zweckmässigerweise bei ungefähr Raumtemperatur.

Falls eine Verbindung der Formel IV, worin $X^1$ ein Sauerstoffatom und $R^4$ und $R^5$ je Wasserstoff bedeuten, erwünscht ist, wird eine Verbindung der Formel XVIII reduziert, und die Alkanoylgruppe aus dem erhaltenen Produkt abgespalten. Die Reduktion erfolgt zweckmässig unter Verwendung eines Borankomplexes, wie Boran/Tetrahydrofuran, Boran/Dimethylsulfid, Boran/N,N-Diäthylanilin oder eines ähnlichen Komplexes. Diese Reduktion wird vorzugsweise in einem inerten organischen Lösungsmittel bei Raumtemperatur oder einer Temperatur unterhalb der Raumtemperatur durchgeführt, beispielsweise unter Verwendung eines Boran/Tetrahydrofuran-Komplexes in Tetrahydrofuran bei ungefähr 0°C bis 20°C. Die Abspaltung der Alkanoylgruppe erfolgt in der weiter oben beschriebenen Weise.

Die Verbindungen der Formel IV, worin $X^1$ ein Sauerstoffatom und $R^{30}$ Aralkyl bedeuten, können beispielsweise durch Abspalten der Estergruppe aus einer Verbindung der Formel IV, worin $X^1$ ein Sauerstoffatom und $R^{30}$ Alkyl bedeuten, und anschliessender Veresterung der erhaltenen Säure zum entsprechenden Aralkylester hergestellt werden.

Die Abspaltung der Estergruppe aus einer Verbindung der Formel IV, worin $X^1$ ein Sauerstoffatom und $R^{30}$ Alkyl bedeuten, zu einer entsprechenden Säure, d.h. einer Verbindung der Formel IV, worin $R^{30}$ Wasserstoff bedeutet, kann beispielsweise durch Umsetzen mit einer Base erfolgen oder, falls die Alkylgruppe die tert.Butylgruppe ist, durch Behandlung mit einer Säure. Geeignete Basen, welche zu diesem Zweck verwendet werden können, sind Alkalimetallhydroxide, z.B. Natriumhydroxid, Kaliumhydroxid und dergleichen, und Ammoniumhydroxid. Die Umsetzung kann bei einer Temperatur zwischen etwa Raumtemperatur und dem Siedepunkt des Reaktionsgemisches, vorzugsweise bei ungefähr Raumtemperatur durchgeführt werden. Wasserfreie Trifluoressigsäure ist eine speziell geeignete Säure zur Abspaltung der Estergruppe aus einer Verbindung der Formel IV, worin $R^{30}$ tert.Butyl bedeutet, wobei die Umsetzung in diesem Falle zweckmässigerweise bei ungefähr Raumtemperatur erfolgt.

Die Veresterung der so erhaltenen Säure kann nach bekannten Methoden erfolgen, beispielsweise durch Umsetzen mit einem geeigneten Alkohol, z.B. Benzylalkohol und dergleichen, in Gegenwart einer Mineralsäure, z.B. Chlorwasserstoffsäure und dergleichen, oder mit einem geeigneten Diazoalkan, z.B. Phenyldiazomethan und dergleichen.

Die in Verfahrensvariante b) als Ausgangsstoffe eingesetzten Verbindungen der Formel IV, worin $X^1$ die Gruppe -NH- bedeutet, sind bekannt.

Die ebenfalls in Verfahrensvariante b) als Ausgangsstoffe eingesetzten Verbindungen der Formel IV, worin $X^1$ die Gruppe $-NR^{61}-$ bedeutet, worin $R^{61}$ Alkyl oder Aralkyl bedeutet, sind neu und ebenfalls Gegenstand der vorliegenden Erfindung. Sie können hergestellt werden, indem man beispielsweise eine Verbindung der Formel IV, worin $X^1$ die Gruppe -NH- bedeutet, alkyliert oder aralkyliert. Die Alkylierung oder Aralkylierung kann in bekannter Weise

durchgeführt werden. Beispielsweise wird eine Verbindung der Formel IV, worin $X^1$ die Gruppe -NH- bedeutet, mit einem geeigneten Alkylhalogenid, z.B. Methylchlorid, Aethylbromid und dergleichen, oder einem geeigneten Aralkylhalogenid, z.B. Benzylbromid und dergleichen, in Gegenwart einer Base umgesetzt.

Die in Verfahrensvariante b) als Ausgangsstoffe eingesetzten Verbindungen der Formel V sind bekannt oder Analoga bekannter Verbindungen und können in analoger Weise wie die bekannten Verbindungen hergestellt werden.

Die in Verfahrensvariante c) als Ausgangsstoffe eingesetzten Verbindungen der Formel VI sind ebenfalls bekannt oder Analoga bekannter Verbindungen und können in analoger Weise zu den bekannten Verbindungen hergestellt werden. Solche Verbindungen der Formel VI, worin $R^3$ Alkyl bedeutet, entsprechen den Verbindungen der obigen Formel VIa. Die Verbindungen der Formel VI, worin $R^3$ Wasserstoff bedeutet, können in bekannter Weise aus den Verbindungen der Formel VIa durch Basenbehandlung oder, falls $R^{31}$ in Formel VIa tert.Butyl bedeutet, durch Behandlung mit wasserfreier Trifluoressigsäure hergestellt werden. Die Verbindungen der Formel VI, worin $R^3$ Aralkyl bedeutet, können aus den Verbindungen der Formel VI, worin $R^3$ Wasserstoff bedeutet, durch Veresterung in bekannter Weise hergestellt werden, beispielsweise durch Umsetzen mit einem geeigneten Alkohol, z.B. Benzylalkohol und dergleichen, in Gegenwart einer Mineralsäure, z.B. Chlorwasserstoffsäure und dergleichen, oder mit einem geeigneten Diazoalkan, z.B. Phenyldiazomethan und dergleichen.

Die in Verfahrensvariante c) ebenfalls als Ausgangsstoffe eingesetzten Verbindungen der Formel VII sind bekannt oder Analoga bekannter Verbindungen und können in analoger Weise zu den bekannten Verbindungen hergestellt werden.

Die Verbindungen der Formel I und deren vorstehend genannte pharmazeutisch verwendbare Salze sind wertvolle Antihypertensiva. Sie hemmen das Angiotensin umwandelnde Enzym (ACE), welches für die Umwandlung von Angiotensin I zu Angiotensin II verantwortlich ist, und sind deshalb nützlich, einen durch Angiotensin bewirkten Bluthochdruck zu vermindern oder zu lindern.

Die Aktivität der vorliegenden Verbindungen, das Angiotensin umwandelnde Enzym in vitro zu hemmen, kann durch den folgenden Test bestimmt werden.

Die verwendete Methode basiert auf einer Methode von Cushman und Cheung (Biochem. Pharmacol., 20, 1637-1648) unter Berücksichtigung der Modifikation von Hayakari et al. (Anal. Biochem., 84, 361-369). Das Substrat (Hippuryl-Histidyl-Leucin, 2 mM) wird in Abwesenheit oder Gegenwart verschiedener Konzentrationen der Testverbindungen in Kaliumphosphatpuffer (pH 8,3; 100 mM), welcher Natriumchlorid (300 mM) enthält, mit Angiotensin umwandelndem Enzym während 24 Minuten bei 37°C (Gesamtwert 500 µl) inkubiert. (Falls die Testsubstanz ein Ester ist, so ist angezeigt, diesen mittels Schweineleberesterase vor Ausführung des Tests zu spalten). Die Reaktion wird durch Zugabe von 3 ml Kaliumphosphatpuffer (pH 8,3; 200 mM) bei 0°C abgebrochen. Man versetzt anschliessend mit 2,4,6-Trichlor-s-triazin (3%) in 1,5 ml Dioxan und schüttelt die erhaltene Mischung, bis der gelbe Chromophor voll entwickelt ist. Die Proben werden anschliessend zentrifugiert, um allenfalls entstandenen Festkörper abzutrennen. Der durch Reaktion von 2,4,6-Trichlor-s-triazin mit freier Hippursäure gebildete gelbe Chromophor wird spektrophotometrisch bei 382 nm ausgemessen. Der $IC_{50}$-Wert ist diejenige Konzentration einer Testverbindung, welche die Spaltung von Hippuryl-Histidyl-Leucin mittels Angiotensin umwandelndem Enzym unter den oben genannten Bedingungen um 50% reduziert.

Die nachfolgende Tabelle enthält die Resultate, welche mit repräsentativen Vertretern der Verbindungen der Formel I im vorhergehend beschriebenen Test erhalten wurden:

Tabelle

| Verbindung | $IC_{50}$ (nM) |
|---|---|
| A | 9,6 |
| B | 5,0 |
| C | 2,4 |
| D | 14,0 |

Verbindung A: Octahydro-9(S)-phosphonomethyl-10-oxo-6H-pyridazo[1,2-a][1,2]diazepine-1(S)-carbonsäure

Verbindung B: Dinatriumsalz von 9(S)-[[Hydroxy(phenäthyl)-phosphinyl]amino]-octahydro-10-oxo-6H-pyridazo-[1,2-a][1,2]diazepin-1(S)-carbonsäure

Verbindung C: Dikaliumsalz von 9(S)-[Hydroxy(4-phenylbutyl)-phosphinyl]-octahydro-10-oxo-6H-pyridazo[1,2-a][1,2]diazepin-1(S)-carbonsäure

Verbindung D: 9(S)-[[(Hydroxy)[4-(1-iminoäthylamino)phenäthyl]phosphinyl]methyl]-6,10-dioxo-6H-pyridazo[1,2-a][1,2]diazepin-1(S)-carbonsäure

Die Verbindungen der Formel I und deren vorstehend genannte pharmazeutisch verwendbare Salze können in Form pharmazeutischer Präparate, welche diese Verbindungen zusammen mit geeigneten pharmazeutischen Trägerstoffen enthalten, als Arzneimittel verwendet werden. Als Trägerstoffe kommen dabei für die enterale, beispielsweise orale, oder parenterale Verabreichung geeignete organische oder

anorganische Trägerstoffe in Frage, beispielsweise Wasser, Gelatine, Gummi arabicum, Lactose, Stärke, Magnesiumstearat, Talk, pflanzliche Oele, Polyalkylenglykole, Vaseline und dergleichen. Die pharmazeutischen Präparate können in fester Form z.B. als Tabletten, Dragées, Suppositorien oder Kapseln, oder in flüssiger Form, z.B. als Lösungen, Suspensionen oder Emulsionen, verarbeitet werden. Die pharmazeutischen Präparate können üblichen pharmazeutischen Behandlungen, wie Sterilisieren, unterworfen werden und/oder Zusatzstoffe, wie Konservierungsmittel, Stabilisierungsmittel, Netzmittel oder Emulgiermittel, Salze zum Variieren des osmotischen Druckes oder Puffer enthalten. Die pharmazeutischen Präparate können auch noch andere therapeutisch wertvolle Verbindungen enthalten.

Die Verbindungen der Formel I und deren vorstehend genannte pharmazeutisch verwendbare Salze können Erwachsenen in täglichen Dosen von etwa 0,1-100 mg, vorzugsweise etwa 1-50 mg, pro kg Körpergewicht verabreicht werden. Die tägliche Dosis kann in einer einzigen Dosis oder Teildosen verabreicht werden. Es sei an dieser Stelle betont, dass die oben genannten Dosierungsbereiche nur als Beispiele angegeben sind, und dass sie nach oben oder unten variiert werden können, in Abhängigkeit von Faktoren, wie den spezifischen Eigenschaften der zu verabreichenden Verbindung oder des zu verabreichenden Salzes, der Art der Verabreichung, der Schwere der zu behandelnden Krankheit und dem Zustand des Patienten, wie er vom behandelnden Arzt festgestellt wird.

Die folgenden Beispiele veranschaulichen die vorliegende Erfindung.

## Beispiel 1

1 g Methyl 9(S)-brommethyl-octahydro-6,10-dioxo -6H-pyridazo[1,2-a][1,2]diazepin-1(S)-carboxylat wurden in

3 ml Trimethylphosphit gelöst, und die Lösung 20 Stunden zum Rückfluss erhitzt. Ueberschüssiges Trimethylphosphit wurde unter vermindertem Druck entfernt, und der Rückstand an Silicagel mit Methanol/Aethylacetat als Eluierungsmittel chromatographiert. Es wurden 720 mg Methyl 9(S)-dimethoxy-phosphinylmethyl-octahydro -6,10-dioxo-6H-pyridazo[1,2-a]-[1,2]diazepin-1(S) -carboxylat in Form eines schwach gelben Oels erhalten.

NMR: δ(300 MHz, $CDCl_3$): 5.45 (1H, m); 4.6 (1H, m); 3.75 (9H, s und zwei t); 3.4 (1H, m); 3.1 (1H, m); 2.85 (1H, m); 2.25-2.55 (4H, m); 1.65-1.95 (5H, m).

Das als Ausgangsmaterial eingesetzte Methyl 9(S)-brommethyl-octahydro -6,10-dioxo-6H-pyridazo[1,2-a][1,2]diazepin -1(S)-carboxylat wurde wie folgt hergestellt:

Eine Lösung von 10 g (0,0313 Mol) 1-Benzyloxycarbonyl-S--piperazinsäure-tert.butylester in 100 ml Methanol wurde bei Raumtemperatur und Atmosphärendruck über 5%-igem Palladium auf Kohle hydriert. Der Katalysator wurde abfiltriert, und das Filtrat zur Trockene eingedampft. Der zurückgebliebene rohe Piperazinsäure-t-butylester wurde in 100 ml Dioxan aufgenommen, und die auf 0°C abgekühlte Lösung mit einer Lösung von 3,94 g (0,0313 Mol) α-Methylenglutarsäurean-hydrid in 100 ml Dioxan versetzt. Das Gemisch wurde 18 Stunden bei 20°C gerührt, und das Lösungsmittel durch Abdampfen entfernt. Der Rückstand wurde zwischen Methyl--tert.butyläther und gesättigter Natriumbiarbonatlösung verteilt. Die wässrige Phase wurde mit Salzsäure angesäuert und mit Methylenchlorid extrahiert, wobei man 8,34 g (85%) 3(S)-tert.Butoxycarbonyl-hexahydro-α-methylen -δ-oxo-1--pyridazinpentansäure in Form von weissen Kristallen erhielt, Schmelzpunkt 96-99°C. 5,0 g (16 mMol) dieser Säure wurden in 350 ml Tetrahydrofuran aufgenommen, und die Lösung auf 0°C gekühlt. 3,75 g (18 mMol) Phosphorpentachlorid wurden zugegeben, und das Gemisch 1 Stunde bei 0°C und 18

Stunden bei 20°C gerührt. Das Lösungsmittel wurde abgedampft, und der Rückstand zwischen Aethylacetat und gesättigter Natriumbicarbonatlösung verteilt. Die organische Phase wurde eingedampft, und der Rückstand an Silicagel mit Aethylacetat/n-Hexan als Eluierungsmittel chromatographiert. Es wurden dabei 3,7 g (79%) tert.Butyl octahydro-9-methylen -6,10-dioxo-6H-pyridazo[1,2-a][1,2]diazepin -1(S)-carboxylat als weisser Festkörper erhalten, Schmelzpunkt 105-106°C (aus Hexan).

10 g tert.Butyl octahydro-9-methylen-6,10-dioxo -6H-pyridazo[1,2-a][1,2]diazepin-1(S)-carboxylat wurden 3 Stunden bei 20°C mit 40 ml Trifluoressigsäure gerührt. Das Gemisch wurde eingedampft, und das erhaltene Oel mit Diäthyläther versetzt, wobei 7,6 g Octahydro-9-methylen- -6,10-dioxo -6H-pyridazo[1,2-a][1,2]diazepin-1(S)-carbonsäure als weisser Festkörper erhalten wurden, Schmelzpunkt 169-172°C.

7,6 g Octahydro-9-methylen -6,10-dioxo-6H-pyridazo-[1,2-a][1,2]diazepin-1(S)-carbonsäure wurden in 200 ml Aethylacetat suspendiert, und die Suspension während der Zugabe von 100 ml einer ätherischen Diazomethanlösung bei 0°C gerührt. Nach 30 Minuten wurde das überschüssige Diazomethan durch tropfenweise Zugabe von Essigsäure zersetzt. Das Gemisch wurde mit wässriger Natriumbicarbonatlösung gewaschen, über Natriumsulfat getrocknet und eingedampft, wobei 5,17 g Methyl octahydro -9-methylen-6,10-dioxo-6H- -pyridazo[1,2-a][1,2] diazepin-1(S)-carboxylat als weisser Festkörper erhalten wurden, Schmelzpunkt 73-75°C (aus n-Hexan).

In eine gerührte Lösung von 5 g Methyl octahydro -9-methylen-6,10-dioxo-6H-pyridazo[1,2-a][1,2]diazepin -1(S)-carboxylat in 125 ml Methylenchlorid wurde während 4 Stunden bei 20°C Bromwasserstoff eingeleitet. Das Gemisch wurde mit gesättigter wässriger Natriumbicarbonatlösung

gewaschen, getrocknet und eingedampft. Der Rückstand wurde an Silicagel mit Aethylacetat/n-Hexan als Eluierungsmittel chromatographiert. Dabei wurden 5,03 g Methyl 9(S)-brommethyl-octahydro -6,10-dioxo-6H-pridazo[1,2-a][1,2]diazepin--1(S)-carboxylat als weisser Festkörper erhalten, Schmelzpunkt 82-83°C (aus Aethylacetat/n-Hexan).

## Beispiel 2

700 mg Methyl 9(S)-dimethoxyphosphinylmethyl-octahydro -6,10-dioxo-6H-pyridazo[1,2-a][1,2]diazepin-1(S)-carboxylat wurden in 15 ml trockenem Tetrahydrofuran gelöst, und die Lösung unter einer Stickstoffatmosphäre bei 0°C gerührt, während 4 ml einer 0,5 molaren Lösung von Boran in Tetrahydrofuran innerhalb von 5 Minuten tropfenweise zugegeben wurde. Das Rühren wurde während 30 Minuten bei 0°C und während weiteren 3 Stunden bei Raumtemperatur fortgesetzt. Das Gemisch wurde mit 75 ml Methylenchlorid verdünnt und gekühlt, während 40 ml 2N Salzsäure vorsichtig zugegeben wurden. Das Rühren wurde für 30 Minuten fortgesetzt, das Gemisch danach mit Natriumcarbonat auf pH 9 eingestellt, und die Phasen getrennt. Die wässrige Phase wurde mit Methylenchlorid gewaschen, und die vereinigten organischen Extrakte über wasserfreiem Natriumsulfat getrocknet und eingedampft. Der Rückstand wurde an Silicagel mit Methanol/Aethylacetat als Eluierungsmittel chromatographiert. Dabei wurden 510 mg Methyl 9(S)-dimethoxyphosphinylmethyl-octahydro -10-oxo-6H--pyridazo[1,2-a][1,2]diazepin-1(S)-carboxylat als farbloses Oel erhalten.

| | |
|---|---|
| Analyse für | $C_{14}H_{25}N_2O_6P$: |
| Berechnet | C, 48,27; H, 7,23; N, 8,04% |
| Gefunden | C, 48,47; H, 7,38; N, 7,89%. |

## Beispiel 3

470 mg Methyl 9(S)-dimethoxyphosphinylmethyl-octahydro

-10-oxo-6H-pyridazo[1,2-a][1,2]diazepin-1(S)-carboxylat wurden mit 2 ml Trimethylsilylbromid während 17 Stunden bei Raumtemperatur umgesetzt. Nach Eindampfen wurde der Rückstand in Aceton aufgenommen, und Wasser zugegeben. Eindampfen lieferte 490 mg Methyl octahydro-9(S)-phosphonomethyl -10-oxo-6H-pyridazo[1,2-a][1,2]diazepin -1(S)-carboxylat-hydrobromid als wachsähnlichen Festkörper.

| | |
|---|---|
| Analyse für | $C_{12}H_{21}N_2O_6P{\bullet}HBr$: |
| Berechnet | C, 35,93; H, 5,52; N, 6,98% |
| Gefunden | C, 36,26; H, 5,30; N, 6,92%. |

## Beispiel 4

450 mg Methyl octahydro-9(S)-phosphonomethyl -10-oxo-6H--pyridazo[1,2-a][1,2]diazepin -1(S)-carboxylat-hydrobromid wurden in 6 ml 350 mg Natriumhydroxid enthaltendem Wasser aufgenommen, und das Gemisch während 4 Stunden bei 20°C stehen gelassen. Die Lösung wurde mit Methylenchlorid gewaschen, die wässrige Phase mit einem gleichen Volumen Methanol verdünnt und auf eine Kolonne von Amberlit CG 120 Kunstharz verbracht. Eluieren mit wässrigem Methanol und Eindampfen des Eluats lieferte nach Anreiben mit Diäthyläther 360 mg Octahydro-9(S)-phosphonomethyl -10-oxo-6H--pyridazo[1,2-a][1,2]diazepin -1(S)-carbonsäure-hydrobromid als amorphen Puder.

| | |
|---|---|
| Analyse für | $C_{11}H_{19}N_2O_6P{\bullet}HBr$: |
| Berechnet | C, 34,13; H, 5,21; N, 7,24% |
| Gefunden | C, 33,91; H, 5,14; N, 7,04%. |

## Beispiel 5

515 mg Methyl 9(S)-brommethyl-octahydro-6,10-dioxo -6H-pyridazo[1,2-a][1,2]diazepin-1(S)-carboxylat wurden in 1,5 ml Diäthoxymethylphosphin gelöst, und die Lösung unter einer Stickstoffatmosphäre während 7 Stunden auf 120°C

erhitzt. Nach Abdampfen wurde der Rückstand an Silicagel mit Aethylacetat als Eluierungsmittel chromatographiert. Dabei wurden 480 mg Methyl 9(S)-[(äthoxy)methylphosphinyl]methyl] -ocatahydro-6,10-dioxo-6H-pyridazo[1,2-a][1,2]diazepin-1(S) -carboxylat als farbloser Gummi erhalten.

MS: m/e 360 ($M^+$).

## Beispiel 6

470 mg Methyl 9(S)-[[(äthoxy)methylphosphinyl]methyl] -octahydro-6,10-dioxo-6H-pyridazo[1,2-][1,2]diazepin -1(S)-carboxylat wurden bei 20°C mit 2 ml Trimethylsilylbromid 17 Stunden gerührt. Nach Eindampfen wurde der Rückstand in Aceton aufgenommen, mit Wasser behandelt und nochmals eingedampft. Der Rückstand wurde in 6 ml 350 mg Natriumhydroxid enthaltendem Wasser gelöst, und die Lösung 3 Stunden bei 20°C stehen gelassen und danach mit Methylenchlorid gewaschen. Die wässrige Phase wurde mit einem gleichen Volumen Methanol verdünnt und danach auf eine Kolonne von Amberlit CG 120 Kunstharz verbracht. Eluieren mit wässrigem Methanol lieferte 250 mg Octahydro-9(S)- -[[(hydroxy)methylphosphinyl]methyl]-6,10-dioxo -6H-pyridazo[1,2-a][1,2]diazepin-1(S)-carbonsäure als weisses Lyophilisat.

| | |
|---|---|
| Analyse für | $C_{12}H_{19}N_2O_6P$: |
| Berechnet | C, 45,3; H, 6,0; N, 8,8 % |
| Gefunden | C, 45,4; H, 5,6; N, 8,55%. |

## Beispiel 7

2,5 g Methyl 9(RS)-brom-octahydro-6,10-dioxo -6H-pyridazo[1,2-a][1,2]diazepin-1(S)-carboxylat wurden mit 15 ml Triäthylphosphit während 12 Stunden zum Rückfluss erhitzt. Eindampfen gefolgt von Chromatographie an Silicagel mit Methanol/Aethylacetat als Eluierungsmittel lieferte 1,4 g

Methyl 9(S)-diäthoxyphosphinyl-octahydro -6,10-dioxo-6H--pyridazo[1,2-a][1,2]diazepin-1(S)-carboxylat als leicht gelbliches Oel.

NMR: δ(300 MHz, $CDCl_3$): 5.4 (1H, m); 4.6 (1H, m); 4.3 (2H, m); 4.1 (2H, m); 3.75 (3H, s); 3.45 (1H, m); 3.25 (1H, m); 2.8 (1H, m); 2.45 (3H, m); 2.25 (1H, m); 1.9 (1H, m); 1.65 (2H, m); 1.3 (6H, m).

## Beispiel 8

330 mg Methyl 9(S)-diäthoxyphosphinyl-octahydro -6,10-dioxo-6H-pyridazo[1,2-a][1,2]diazepin-1(S)-carboxylat wurden mit 2 ml Trimethylsilylbromid versetzt, und das Gemisch während 17 Stunden bei 20°C gerührt. Die Lösung wurde zur Trockene eingedampft, und der Rückstand in Aceton aufgenommen und mit Wasser vesetzt. Nach Eindampfen wurde der Rückstand in 5 ml 200 mg Natriumhydroxid enthaltendem Wasser gelöst, und die Lösung 4 Stunden bei 20°C stehen gelassen. Nach Waschen mit Methylenchlorid wurde die wässrige Phase auf eine Amberlit CG 120 Kunstharzsäule verbracht, welche dann mit Wasser eluiert wurde. Das Eluat wurde eingedampft, und der Rückstand mit Diäthyläther angerieben, wobei 110 mg Octahydro-9(S)-phosphono -6,10-dioxo--6H-pyridazo[1,2-a][1,2]diazepin-1(S)-carbonsäure als amorpher Puder erhalten wurden.

NMR: δ(400 MHz, $CD_3OD$): 5.4 (1H, m); 4.55 (1H, m): 3.55 (1H, m); 3.25 (1H, m); 2.95 (1H, m): 2.4 (2H, m); 2.3 (2H, m); 1.95 (1H, m); 1.6-1.8 (2H, m).

## Beispiel 9

2,8 g Methyl 9(S)-diäthoxyphosphinyl-octahydro -6,10-dioxo-6H-pyridazo[1,2-a][1,2]diazepin -1(S)-carboxylat wurden in 40 ml Aethanol gelöst, und die Lösung mit einer Lösung von 305 mg Natriumhydroxid in 10 ml Wasser versetzt.

Nach 30 Minuten bei 20°C wurde die Lösung eingedampft. Der Rückstand wurde in gesättigter Kochsalzlösung aufgenommen, mit Diäthyläther gewaschen, mit 2N Salzsäure angesäuert und mit Methylenchlorid extrahiert. Die Methylenchloridextrakte wurden über wasserfreiem Natriumsulfat getrocknet und eingedampft. Der Rückstand wurde aus Aethanol/Diäthyläther/n-Hexan umkristallisiert, wobei 1,4 g 9(S)-Diäthoxyphosphinyl-octahydro -6,10-dioxo-6H-pyridazo[1,2-a][1,2]diazepin-1(S)-carbonsäure als weisser Festkörper erhalten wurden, Schmelzpunkt 187-189°C (Zersetzung).

| | |
|---|---|
| Analyse für | $C_{14}H_{23}N_2O_7P$: |
| Berechnet | C, 46,41; H, 6,4; N, 7,73% |
| Gefunden | C, 46,7; H, 6,42; N, 7,79%. |

## Beispiel 10

700 mg Methyl 9(S)-dimethoxyphosphinylmethyl-octahydro -6,10-dioxo-6H-pyridazo[1,2-a][1,2]diazepin-1(S)-carboxylat wurden in 10 ml 2-Butanon gelöst, und dann wurden 320 mg Lithiumthiocyanat-monohydrat zugegeben. Das Gemisch wurde 24 Stunden zum Rückfluss erhitzt und danach eingedampft. Der Rückstand wurde in 5 ml Methanol gelöst, und 3,9 ml 1N Natriumhydroxid-Lösung wurden zugegeben. Nach 5 Minuten bei 20°C wurde die Lösung auf eine Amberlit CG 120 Kunstharzsäule verbracht. Eluieren mit wässrigem Methanol lieferte 620 mg Octahydro-9(S)-[[hydroxy(methoxy)phosphinyl]methyl] -6,10-dioxo-6H-pyridazo[1,2-a][1,2]diazepin-1(S)-carbonsäure als weissen Festkörper, Schmelzpunkt 80°C (Zersetzung) (nach Anreiben mit Diäthyläther).

| | |
|---|---|
| Analyse für | $C_{12}H_{19}N_2O_7P$: |
| Berechnet | C, 43,12; H, 5,73; N, 8,38% |
| Gefunden | C, 43,31; H, 5,57; N, 8,20%. |
| (wasserfrei) | |

## Beispiel 11

In analoger Weise wie in Beispiel 1 beschrieben, wurden aus 500 mg Methyl 9(S)-brommethyl-octahydro-6,10-dioxo -6H-pyridazo[1,2-a][1,2]diazepin-1(S)-carboxylat und 1 ml Triäthylphosphit 300 mg Methyl 9(S)-diäthoxyphosphinylmethyl-octahydro-6,10-dioxo -6H-pyridazo[1,2-a][1,2]diazepin-1(S)-carboxylat erhalten, das in Form eines farblosen Gummis isoliert wurde.

NMR: δ($CDCl_3$, 300 MHz): 5.45 (1H, m); 4.6 (1H, m); 4.1 (4H, m); 3.8 (3H, s); 3.4 (1H, m); 3.1 (1H, m); 2.85 (1H, m); 2.25-2.55 (4H, m), 1.6-2.0 (5H, m); 1.3 (6H, m).

## Beispiel 12

In analoger Weise wie in Beispiel 8 beschrieben, wurden aus 300 mg Methyl 9(S)-diäthoxyphosphinylmethyl-octahydro -6,10-dioxo-6H-pyridazo[1,2-a][1,2]diazepin-1(S)-carboxylat 250 mg Octahydro-9(S)-phosphonomethyl-6,10-dioxo -6H-pyridazo[1,2-a][1,2]diazepin-1(S)-carbonsäure in Form eines weissen Lyophilisates erhalten.

NMR: δ($D_2O$, 300 MHz): 5.35 (1H, m); 4.4 (1H, m); 3.3 (1H, m); 3.1 (2H, m); 2.45 (1H, m); 2.3 (2H, m); 2.15 (1H, m); 1.95 (2H, m); 1.75 (3H, m).

## Beispiel 13

In analoger Weise wie in Beispiel 5 beschrieben, wurden aus 200 mg Methyl 9(S)-brommethyl-octahydro-6,10-dioxo -6H-pyridazo[1,2-a][1,2]diazepin-1(S)-carboxylat und 700 mg Diäthoxy-(3-phenylpropyl)phosphin 110 mg Methyl 9(S)--[[äthoxy(3-phenylpropyl)phosphinyl]methyl] -octahydro-6,10--dioxo-6H-pyridazo[1,2-a][1,2]diazepin -1(S)-carboxylat als farbloser Gummi erhalten.

MS: m/e E.I. 464 ($M^+$).

## Beispiel 14

100 mg Methyl 9(S)-[äthoxy(3-phenylpropyl)phosphinyl]-methyl -octahydro-6,10-dioxo-6H-pyridazo[1,2-a][1,2]diazepin -1(S)-carboxylat wurden in 1 ml Methanol gelöst, und die Lösung mit 1 ml 50 mg Natriumhydroxid enthaltendem Wasser versetzt. Die Lösung wurde 5 Stunden bei 20°C stehen gelassen und danach auf eine Amberlit CG 120 Kunstharzsäule verbracht. Eluieren mit wässrigem Methanol und Eindampfen des Eluats lieferte einen Gummi, der in 1 ml Acetonitril gelöst wurde. 0,5 ml Trimethylsilylbromid wurden zur Lösung gegeben, die dann 17 Stunden bei 20°C gerührt und danach eingedampft wurde. Der Rückstand wurde in Aceton gelöst und mit Wasser versetzt. Eindampfen lieferte 60 mg 9-(S)-[[Hydroxy(3-phenylpropyl)phosphinyl]methyl] -octahydro-6,10-dioxo-6H-pyridazo[1,2-a][1,2]diazepin -1(S)-carbonsäure als leicht gelblichen Schaum.

NMR: δ($CD_3OD$, 300 MHz): 7.2 (5H, m); 5.3 (1H, m); 4.45 (1H, m); 3.5 (1H, m); 3.1 (1H, m); 2.9 (1H, m); 2.7 (2H, t); 2.35 (2H, m); 2.2 (2H, m); 1.85 (4H, m); 1.7 (5H, m).

## Beispiel 15

1,2 g Benzyl 9(S)-amino-octahydro-10-oxo-6H-pyridazo [1,2-a][1,2]diazepin-1(S)-carboxylat wurden in 25 ml Methylenchlorid gelöst. 0,56 ml Triäthylamin wurden zugegeben, gefolgt von einer Lösung von 0,79 g Benzyl phenäthylphosphorchloridat in 5 ml Methylenchlorid. Das Gemisch wurde 4 Stunden bei 20°C gerührt, mit Wasser gewaschen, über wasserfreiem Natriumsulfat getrocknet und eingedampft. Der Rückstand wurde an Silicagel mit Aethylacetat/n-Hexan als Eluierungsmittel chromatographiert. Dabei wurden 550 mg Benzyl 9(S)-[[benzyloxy(phenäthyl)phosphinyl]amino] -octahydro-10-oxo-6H-pyridazo[1,2-a][1,2]diazepin -1(S)-carboxylat

als leicht gelblicher Gummi erhalten.

MS: m/e 576 $(M+H)^+$.

## Beispiel 16

250 mg Benzyl 9(S)-[[benzyloxy(phenäthyl)phosphinyl]-amino] -octahydro-10-oxo-6H-pyridazo[1,2-a][1,2]diazepin -1(S)-carboxylat und 73 mg Natriumbicarbonat wurden in 100 ml 25%-igem wässrigem Aethanol gelöst, und das Gemisch während 17 Stunden bei Atmosphärendruck über 10% Palladium auf Kohle hydriert. Der Katalysator wurde abfiltriert, und das Filtrat eingedampft. Der Rückstand wurde in Wasser aufgenommen und gefriergetrocknet, wobei 145 mg des Dinatriumsalzes von 9(S)-[[Hydroxy(phenäthyl)phosphinyl]-amino] -octahydro-10-oxo-6H-pyridazo[1,2-a][1,2]diazepin -1(S)-carbonsäure als weisses Lyophilisat erhalten wurden.

| | |
|---|---|
| Analyse für | $C_{18}H_{24}N_3O_5PNa_2 \cdot 2H_2O$: |
| Berechnet | C, 43,82; H, 6,12; N, 8,51% |
| Gefunden | C, 43,69; H, 5,9; N, 8,6 %. |

## Beispiel 17

30 mg 80%-iges Natriumhydrid wurden zu einer gerührten Lösung von 298 mg tert.Butyl octahydro-9(S)-hydroxy-6,10--dioxo -6H-pyridazo[1,2-a][1,2]diazepin-1(S)-carboxylat in 4 ml Tetrahydrofuran gegeben, und das Gemisch 5 Minuten bei Raumtemperatur gerührt. Eine Lösung von 297 mg Dibenzylchlorophosphonat in 3 ml Tetrachlorkohlenstoff wurden zugegeben, das Gemisch 1 Stunde bei Raumtemperatur gerührt, und die Lösungsmittel danach durch Eindampfen entfernt. Der Rückstand wurde an Silicagel mit Diäthyläther/Methanol (19:1) als Eluierungsmittel chromatographiert, wobei 95 mg tert.Butyl 9(S)-(dibenzylphosphonoxy)-octahydro -6,10-dioxo--6H-pyridazo[1,2-a][1,2] diazepin-1(S)-carboxylat als leicht

gelblicher Gummi erhalten wurden.

Analyse für $C_{28}H_{35}N_2O_8P$:
Berechnet C, 60,2; H, 6,3; N, 5,0%
Gefunden C, 59,9; H, 6,2; N, 4,9%.

Das als Ausgangsmaterial eingesetzte tert.Butyl octahydro-9(S)-hydroxy-6,10-dioxo-6H-pyridazo [1,2-a][1,2]diazepin-1(S)-carboxylat wurde wie folgt hergestellt:

9,6 g 1-Benzyl 3-tert.butyl-hexahydropyridazin-1,3(S)-dicarboxylat wurden bei 8-10°C mit 50 ml Toluol und 70 ml einer 10%-igen wässrigen Natriumbicarbonatlösung gerührt, während eine Lösung des Säurechlorids aus 89,1 g 2-Acetoxy-4-benzyloxycarbonylbutansäure in 45 ml Toluol zugegeben wurde. Das Gemisch wurde 1 Stunde bei Raumtemperatur gerührt, und die organische Phase abgetrennt. Chromatographie an Florisil mit Toluol als Eluierungsmittel lieferte 15,3 g 1-Benzyl 3-tert.butyl 2-[2(S)-acetoxy -4-(benzyloxycarbonyl)butyryl]-hexahydro -1,3(S)-pyridazindicarboxylat als farblosen Gummi.

14,5 g 1-Benzyl 3-tert.butyl 2-[2(S)-acetoxy-4-(benzyloxycarbonyl)butyryl]-hexahydro -1,3(S)-pyridazindicarboxylat in 150 ml Dimethylformamid wurden über 0,5 g 10%-igem Palladium auf Kohle bei Raumtemperatur und Atmosphärendruck hydriert. Der Katalysator wurde abfiltriert, und die Lösungsmittel danach durch Eindampfen entfernt. Der Rückstand wurde in 150 ml Methylenchlorid aufgenommen, und die Lösung gekühlt und unter einem langsamen Strom von Stickstoff während der Zugabe von 2,97 g Thionylchlorid gerührt. Die Lösung wurde 2 Stunden bei Raumtemperatur gerührt und danach mit 10%-iger wässriger Kaliumbicarbonatlösung und gesättigter Natriumchloridlösung gewaschen, über Magnesiumsulfat getrocknet und eingedampft. Der Rückstand wurde mit Diäthyläther/Aethylacetat (1:1) als Eluierungsmittel an Silicagel chromatographiert. Dabei wurden 5,7 g tert.Butyl

9(S)-acetoxy-octahydro-6,10-dioxo -6H-pyridazo[1,2-a][1,2]-diazepin-1(S)-carboxylat als weisser Festkörper erhalten, Schmelzpunkt 80-81°C (aus Diäthyläther/n-Hexan).

Eine Lösung von 0,68 g tert.Butyl 9(S)-acetoxy-octahydro -6,10-dioxo-6H-pyridazo[1,2-a][1,2]diazepin-1(S)-carboxylat in 5 ml Aethanol wurde während 1 Stunde bei Raumtemperatur mit 10 ml einer 0,4N wässrigen Natriumhydroxidlösung versetzt. Das Lösungsmittel wurde abgedampft, und der Rückstand zwischen Wasser und Methylenchlorid verteilt. Die organische Phase wurde abgetrennt und eingedampft, wobei 0,52 g tert.Butyl octahydro-9(S)-hydroxy-6,10-dioxo-6H--pyridazo[1,2-a] [1,2]diazepin-1(S)-carboxylat als weisser Festkörper erhalten wurde, Schmelzpunkt 137-138°C (aus Diäthyläther).

| | |
|---|---|
| Analyse für | $C_{29}H_{37}N_2O_7P$: |
| Berechnet | C, 56,4; H, 7,4; N, 9,4 % |
| Gefunden | C, 56,2, H, 7,5; N, 9,35%. |

## Beispiel 18

0,15 g 80%-iges Natriumhydrid wurde zu einer gerührten Lösung von 1,49 g tert.Butyl octahydro-9(S)-hydroxy-6,10--dioxo-6H-pyridazo[1,2-a] [1,2]diazepin-1(S)-carboxylat in 20 ml Tetrahydrofuran gegeben, und das Gemisch 5 Minuten bei Raumtemperatur gerührt. Eine Lösung von 1,47 g Benzyl phenäthylphosphorchloridat in 10 ml Tetrahydrofuran wurde zugegeben, das Gemisch eine Stunde bei Raumtemperatur gerührt, und die Lösungsmittel danach abgedampft. Der Rückstand wurde mit Diäthyläther/Methanol (19:1) als Eluierungsmittel an Silicagel chromatographiert. Dabei wurden 1,44 g tert.Butyl 9(S)-[benzyloxy(phenäthyl)phosphinyloxy]-octahydro -6,10-dioxo-6H-pyridazo[1,2-a][1,2]diazepin-1(S)-carboxylat als farbloser Gummi erhalten.

Analyse für $C_{29}H_{37}N_2O_7P$:
Berechnet C, 62,6, H, 6,7; N, 5,0%
Gefunden C, 62,7; H, 6,7; N, 5,1%.

## Beispiel 19

0,1 g Triäthylamin und 0,35 g Dibenzylchlorophosphonat in 3,6 ml Tetrachlorkohlenstoff wurden zu einer gerührten und auf 0°C gekühlten Lösung von 0,32 g Benzyl 9(S)-amino--octahydro -10-oxo-6H-pyridazo[1,2-a][1,2]diazepin-1(S)-carboxylat in 5 ml Methylenchlorid gegeben. Das Gemisch wurde 16 Stunden bei Raumtemperatur gerührt, und die Lösungsmittel danach abgedampft. Der Rückstand wurde an Silicagel mit Aethylacetat/n-Hexan (2:1) als Eluierungsmittel chromatographiert. Dabei wurde 0,25 g Benzyl 9(S)-[(dibenzyloxyphosphinyl)amino]-octahydro -10-oxo-6H-pyridazo[1,2-a][1,2]diazepin-1(S)-carboxylat als farbloser Gummi erhalten.

NMR: δ ($CDCl_3$, 300 MHz): 1.3-1.9 (6H, m); 2.0 (1H, m); 2.34 (1H, breit); 2.41 (1H, m); 2.88 (1H, breit); 2.98 (1H, m); 3.13 (1H, m); 4.03 (1H, m); 4.71 (1H, m); 5.0-5.1 (5H, m); 5.14 (1H, d); 5.20 (1H, d); 7.2 -7.5 (15H, m).

## Beispiel 20

Eine Lösung von 1,88 g Benzyl 9(S)-[(dibenzyloxyphosphinyl)amino]-octahydro -10-oxo-6H-pyridazo[1,2-a][1,2-diazepin-1(S)-carboxylat in 20 ml Methanol wurde mit 4,9 ml einer 1N wässrigen Natriumhydroxidlösung 16 Stunden bei Raumtemperatur versetzt. Die Lösung wurde mit 50 ml Wasser verdünnt, und das Volumen danach auf 20 ml reduziert. Der pH-Wert wurde auf 3,5 eingestellt, und die Lösung mit Methylenchlorid extrahiert. Die organische Lösung wurde eingedampft, und der Rückstand an Silicagel chromatographiert, wobei 1,42 g 9(S)-[(Dibenzyloxyphosphinyl)amino]--octahydro -10-oxo-6H-pyridazo[1,2-a][1,2]diazepin-1(S)-carbonsäure als Lyophilisat (aus Benzol) erhalten wurden.

| | |
|---|---|
| Analyse für | $C_{24}H_{30}N_3O_6P$: |
| Berechnet | C, 59,1; H, 6,2; N, 8,6% |
| Gefunden (wasserfrei) | C, 58,95; H, 5,9; N, 8,3%. |

## Beispiel 21

In analoger Weise wie in Beispiel 18 beschrieben, wurde aus 1,1 g tert.Butyl octahydro-9(S)-hydroxy-6,10-dioxo -6H-pyridazo[1,2-a][1,2]diazepin-1(S)-carboxylat und 1,21 g Benzyl 4-phenylbutylphosphorchloridat 0,90 g tert.Butyl 9(S)-[benzyloxy(4-phenylbutyl)phosphinyloxy]-octahydro -6,10-dioxo-6H-pyridazo[1,2-a][1,2]diazepin-1(S)-carboxylat als farbloser Gummi erhalten.

MS: m/e 584 ($M^+$).

## Beispiel 22

Eine Lösung von 0,4 g tert.Butyl 9(S)-[benzyloxy(4--phenylbutyl)phosphinyloxy]-octahydro -6,10-dioxo-6H-pyridazo[1,2-a][1,2]diazepin-1(S)-carboxylat in 40 ml Isopropanol wurde über 10%-igem Palladium auf Kohle während 16 Stunden bei Atmosphärendruck hydriert. Der Katalysator wurde abfiltriert, und das Filtrat eingedampft. Der zurückbleibende Gummi wurde in 10 ml Trifluoressigsäure gelöst, die Lösung 1 Stunde bei 20°C stehen gelassen und danach eingedampft, wobei 0,24 g 9(S)-[Hydroxy(4-phenylbutyl)phosphinyloxy] -octahydro-6,10-dioxo-6H-pyridazo[1,2-a][1,2]-diazepin -1(S)-carbonsäure als farbloser Gummi erhalten wurde.

MS: m/e 439 $(M + H)^+$.

## Beispiel 23

In analoger Weise wie in Beispiel 15 beschrieben, wurde aus 0,8 g Benzyl 9(S)-amino-octahydro-10-oxo -6H-pyridazo-[1,2-a][1,2]diazepin-1(S)-carboxylat und 0,49 g Benzyl benzylphosphorchloridat 0,127 g Benzyl 9(S)-[[benzyloxy-(benzyl)phosphinyl]amino]-octahydro -10-oxo-6H-pyridazo-[1,2-a][1,2]diazepin-1(S)-carboxylat als farbloser Gummi erhalten.

MS: m/e 561 ($M^+$).

## Beispiel 24

In analoger Weise wie in Beispiel 15 beschrieben, wurde aus 1,15 g Benzyl 9(S)-amino-octahydro -10-oxo-6H-pyridazo-[1,2-a][1,2]diazepin-1(S)-carboxylat und 0,73 g Benzyl 3-phenylpropylphosphorchloridat 0,31 g Benzyl 9(S)-[[benzyloxy(3-phenylpropyl)phosphinyl]amino] -octahydro-10-oxo-6H--pyridazo[1,2-a][1,2]diazepin-1(S) -carboxylat als farbloser Gummi erhalten.

MS: m/e 589 ($M^+$).

## Beispiel 25

In analoger Weise wie in Beispiel 15 beschrieben, wurde aus 1,13 g Benzyl 9(S)-amino-octahydro-10-oxo-6H-pyridazo-[1,2-a][1,2] diazepin-1(S)-carboxylat und 0,83 g Benzyl 4-phenylbutylphosphorchloridat 0,33 g Benzyl 9(S)-[[benzyloxy(4-phenylbutyl)-phosphinyl]amino] -octahydro-10-oxo-6H--pyridazo[1,2-a][1,2]diazepin -1(S)-carboxylat als farbloser Gummi erhalten.

MS: m/e 603 ($M^+$).

## Beispiel 26

In analoger Weise wie in Beispiel 16 beschrieben, wurden aus 260 mg Benzyl 9(S)-[[benzyloxy(benzyl)phosphinyl]amino]-octahydro-10-oxo -6H-pyridazo[1,2-a][1,2]diazepin-1(S)-carboxylat 190 mg des Dinatriumsalzes von 9(S)-[[Hydroxy-(benzyl)phosphinyl]amino]-octahydro-10 -oxo-6H-pyridazo-[1,2-a][1,2]diazepin-1(S)-carbonsäure als weisses Lyophilisat erhalten.

MS: m/e 426 $(M+H)^+$.

## Beispiel 27

In analoger Weise wie in Beispiel 16 beschrieben, wurden aus 290 mg Benzyl 9(S)-[[benzyloxy(3-phenylpropyl)- phosphinyl]amino] -octahydro-10-oxo-6H-pyridazo[1,2-a][1,2]diazepin-1(S) -carboxylat 230 mg des Dinatriumsalzes von 9(S)-[[Hydroxy(3-phenylpropyl)phosphinyl]amino]-octahydro -10-oxo-6H-pyridazo[1,2-a][1,2]diazepin-1(S)-carbonsäure als weisses Lyophilisat erhalten.

| | |
|---|---|
| Analyse für | $C_{19}H_{26}N_3O_5P\ Na_2 \cdot 2.2\ H_2O$: |
| Berechnet | C, 46.29; H, 6.21; N, 8.52; P 6.28; $H_2O$ 8.04% |
| Gefunden | C, 46.15; H, 6.40; N, 8.20; P 6.30; $H_2O$ 8.00% |

## Beispiel 28

In analoger Weise wie in Beispiel 16 beschrieben, wurden aus 280 mg Benzyl 9(S)-[[benzyloxy(4-phenylbutyl)phosphinyl]amino]-octahydro -10-oxo-6H-pyridazo[1,2-a][1,2]diazepin-1(S)-carboxylat 200 mg des Dinatriumsalzes von 9(S)-[[Hydroxy(4-phenylbutyl)phosphinyl]amino]-octahydro -10-oxo-6H-pyridazo[1,2-a][1,2]diazepin-1(S)-carbonsäure in Form eines weissen Lyophilisates erhalten.

| | |
|---|---|
| Analyse für | $C_{20}H_{28}N_3O_5P\ Na_2 \cdot 2.1\ H_2O$: |
| Berechnet | C, 47.54; H, 6.42; N, 8.32; $H_2O$ 7.48% |
| Gefunden | C, 47.40; H, 6.30; N, 8.10; $H_2O$ 7.55%. |

## Beispiel 29

3 g Methyl 9(RS)-brom-octahydro-6,10 -dioxo-6H-pyridazo-[1,2-a][1,2]diazepin-1(S)-carboxylat und 8.1 g Diäthyl 5-phenylpentylphosphonit wurden zusammen 12 Stunden auf 150°C erhitzt. Eindampfen und Chromatographie an Silicagel mit Methanol/Aethylacetat als Eluierungsmittel lieferte 1.3 g Methyl 9(S)-[(äthoxy)(5-phenylpentyl)phosphinyl]-octahydro-6,10 -dioxo-6H-pyridazo[1,2-a][1,2]diazepin-1(S)-carboxylat als farblosen Gummi.

MS: m/e 479 $(M+H)^+$.

## Beispiel 30

In analoger Weise wie in Beispiel 29 beschrieben, wurden aus 1 g Methyl 9(RS)-brom-octahydro-6,10 -dioxo-6H-pyridazo-[1,2-a][1,2]diazepin-1(S)-carboxylat und 3 g Diäthyl 4-phenylbutylphosphonit 350 mg Methyl 9(S)-[(äthoxy)(4--phenylbutyl)phosphinyl]-octahydro-6,10 -dioxo-6H-pyridazo-[1,2-a][1,2]diazepin-1(S)-carboxylat als Gummi erhalten.

NMR: δ($CDCl_3$, 300 MHz): 7.20 (SH, m); 5 39 (1H, dd, J = 3, 7Hz); 4.56 (1H, m); 4.08 (2H, m); 3.74 (3H, s); 3.45 (1H, m); 3.20 (1H, m); 2.80 (1H, m); 2.63 (2H, t, J = 7Hz); 2.44 (2H, m); 2.25 (3H, m); 1.90-1.55 (8H, m); 1.30 (3H, t, J = 7.5 Hz).

## Beispiel 31

In analoger Weise wie in Beispiel 29 beschrieben, wurden aus 1 g Methyl 9(RS)-brom-octahydro-6,10 -dioxo-6H-pyridazo-[1,2-a][1,2]diazepin-1(S)-carboxylat und 2.25 g Diäthyl

3-phenylpropylphosphonit 220 mg Methyl 9(S)-[(äthoxy)(3--phenylpropyl)phosphinyl]-octahydro-6,10 -dioxo-6H-pyridazo-[1,2-a][1,2]diazepin-1(S)-carboxylat als Gummi erhalten.

NMR: δ($CDCl_3$, 400 MHz): 7.24 (5H, m); 5.40 (1H, dd, J = 3, 7Hz); 4.60 (1H, m); 4.17 - 3.99 (2H, m); 3.76 (3H, s); 3.44 (1H, m); 3.20 (1H, m); 2.86 (1H, m); 2.72 (2H, m); 2.40 (2H, m); 2.24 (3H, m); 2.10 (1H, m); 1.91 (3H, m); 2.70 (2H, m); 1.30 (3H, t, J = 7.5Hz).

## Beispiel 32

In analoger Weise wie in Beispiel 29 beschrieben, wurden aus 1 g Methyl 9(RS)-brom-octahydro-6,10 -dioxo-6H-pyridazo-[1,2-a][1,2]diazepin-1(S)-carboxylat und 2.6 g Diäthyl 2-phenäthylphosphonit 410 mg Methyl 9(S)-[(äthoxy)(phenäthyl)phosphinyl]-octahydro-6,10-dioxo-6H-pyridazo[1,2-a][1,2] diazepin -1(S)-carboxylat als Gummi erhalten.

MS: m/e 436 ($M^+$).

## Beispiel 33

1,2 g Methyl 9(S)-[(äthoxy)(5-phenylpentyl)phosphinyl]--octahydro -6,10-dioxo-6H-pyridazo[1,2-a][1,2]diazepin -1(S)-carboxylat wurden mit einer Lösung von 1 g Natriumhydroxid in 15 ml Wasser versetzt, und das Gemisch 24 Stunden bei 20°C gerührt. Das Gemisch wurde auf eine Ionenaustauscher-Säule von Sulfonsäure-Kunstharz verbracht und mit 50%-igem Methanol/Wasser eluiert. Das Eluat wurde eingedampft, und der Rückstand an Silicagel mit Chloroform/Methanol/Essigsäure/Wasser (120:14:3:2) als Eluierungsmittel chromatographiert. Das Produkt wurde in 20 ml Acetonitril gelöst, und die Lösung mit 5 ml Trimethylsilylbromid versetzt. Die Lösung wurde 17 Stunden bei 20°C gerührt und danach eingedampft. Der Rückstand wurde in 30 ml Aceton gelöst; danach wurden 20 ml Wasser zugegeben. Nach 2 Stunden

wurde das Gemisch eingedampft, und der Rückstand mit Diäthyläther versetzt, wobei 520 mg 9(S)-[Hydroxy(5-phenylpentyl)phosphinyl]-octahydro-6,10 -dioxo-6H-pyridazo[1,2-a]-[1,2]diazepin-1(S)-carbonsäure als weisser Festkörper erhalten wurden, Schmelzpunkt 208-220°C (aus Methanol).

| | |
|---|---|
| Analyse für | $C_{21}H_{29}N_2O_6P$: |
| Berechnet | C, 57.79; H, 6.70; N, 6.42% |
| Gefunden | C, 57.85; H, 6.68; N, 6.52%. |

## Beispiel 34

In analoger Weise wie in Beispiel 6 beschrieben, wurden aus 300 mg Methyl 9(S)-[(äthoxy)(4-phenylbutyl)phosphinyl]--octahydro-6,10 -dioxo-6H-pyridazo[1,2-a][1,2]diazepin-1(S)--carboxylat 150 mg 9(S)-[Hydroxy(4-phenylbutyl)phosphinyl] -octahydro-6,10-dioxo-6H-pyridazo[1,2-a][1,2]diazepin--1(S)-carbonsäure als weisses Lyophilisat erhalten.

NMR: δ($CD_3OD$, 300 MHz): 7.20 (5H, m); 5.30 (1H, m); 4.45 (1H, m); 3.48 (1H, m); 3.20 (1H, m); 2.82 (1H, m); 2.61 (2H, m); 2.30 (4H, m); 2.10 - 1.50 (9H, m).

## Beispiel 35

In analoger Weise wie in Beispiel 6 beschrieben, wurden aus 210 mg Methyl 9(S)-[(äthoxy)(3-phenylpropyl)phosphinyl]--octahydro-6,10 -dioxo-6H-pyridazo[1,2-a][1,2]diazepin-1(S)--carboxylat 80 mg 9(S)-[Hydroxy(3-phenylpropyl)phosphinyl] -octahydro-6,10-dioxo-6H-pyridazo[1,2-a][1,2]diazepin--1(S)-carbonsäure als amorpher weisser Festkörper erhalten.

| | |
|---|---|
| Analyse für | $C_{19}H_{25}N_2O_6P$: |
| Berechnet | C, 55.88; H, 6.17; N, 6.86% |
| Gefunden | C, 55.66; H, 6.00; N, 6.69%. |

## Beispiel 36

In analoger Weise wie in Beispiel 33 beschrieben, wurden aus 340 mg Methyl 9(S)-[(äthoxy)(phenäthyl)phosphinyl]-octahydro-6,10 -dioxo-6H-pyridazo[1,2-a][1,2]diazepin-1(S)-carboxylat 100 mg 9(S)-[Hydroxy(phenäthyl)phosphinyl] -octahydro-6,10- -dioxo-6H-pyridazo[1,2-a][1,2]diazepin-1(S)-carbonsäure als leicht bräunliches Lyophilisat erhalten.

NMR: δ($CD_3OD$, 300 MHz): 3.24 (5H, m); 5.32 (1H, m); 4.46 (1H, m); 3.50 (1H, m); 3.15 (1H, m); 2.90 (3H, m); 2.50 - 2.15 (6H, m); 1.90 (1H, m); 1.65 (2H, m).

## Beispiel 37

In analoger Weise wie in Beispiel 5 beschrieben, wurden aus 1 g Methyl 9(S)-brommethyl-octahydro -6,10-dioxo-6H- -pyridazo[1,2-a][1,2]diazepin-1(S)-carboxylat und 2 g Diäthyl 2-phenäthylphosphonit 630 mg Methyl 9(S)-[[äthoxy-(phenäthyl)phosphinyl]methyl]octahydro-6,10 -dioxo-6H-pyridazo[1,2-a][1,2]diazepin-1(S)-carboxylat als leicht gelblicher Gummi erhalten.

NMR: δ($CDCl_3$, 300 MHz); 7.26 (5H, m); 5.42 (1H, dd, J = 3.7Hz); 4.60 (1H, m); 4.05 (2H, m); 3.73 (2H, s); 3.40 (1H, m); 3.18 (1H, m); 2.90 (3H, m); 2.35 (4H, m); 2.05 (2H, m); 1.87 (2H, m); 1.65 (3H, m); 1.30 (3H, t, J = 7.5Hz).

## Beispiel 38

In analoger Weise wie in Beispiel 6 beschrieben, wurden aus 630 mg Methyl 9(S)-[[äthoxy(phenäthyl)phosphinyl]-methyl]octahydro-6,10 -dioxo-6H-pyridazo[1,2-a][1,2]diazepin-1(S)-carboxylat 430 mg 9(S)-[[Hydroxy(phenäthyl)phosphinyl]methyl]octahydro-6,10 -dioxo-6H-pyridazo[1,2-a][1,2]-diazepin-1(S)-carbonsäure als weisses Lyophilisat erhalten.

Analyse für $C_{19}H_{25}N_2O_6P\bullet0.5H_2O$:
Berechnet C, 54.67, H, 6.27; N, 6.71; $H_2O$ 2.16%
Gefunden C, 54.55; H, 6.12; N, 6.40; $H_2O$ 1.98%.

## Beispiel 39

In analoger Weise wie in Beispiel 2 beschrieben, wurden aus 1.34 g Methyl 9(S)-[[äthoxy(phenäthyl)phosphinyl]-methyl]octahydro-6,10 -dioxo-6H-pyridazo[1,2-a][1,2]diazepin-1(S)-carboxylat 350 mg Methyl 9(S)-[[äthoxy(phenäthyl)-phosphinyl]methyl]octahydro-10 -oxo-6H-pyridazo[1,2-a][1,2]-diazepin-1(S)-carboxylat als farbloses Oel erhalten.

MS: m/e 436 ($M^+$).

## Beispiel 40

In analoger Weise wie in Beispiel 6 beschrieben, wurden aus 330 mg Methyl 9(S)-[[äthoxy(2-phenäthyl)phosphinyl]-methyl]octahydro-10 -oxo-6H-pyridazo[1,2-a][1,2]diazepin-1(S)-carboxylat 240 mg 9(S)-[[Hydroxy(2 -phenäthyl)phosphinyl]methyl]octahydro-10-oxo-6H -pyridazo[1,2-a][1,2]diazepin-1(S)-carbonsäure als leicht gelbliches Lyophilisat aus Essigsäure erhalten.

NMR: δ($CD_3OD$, 300 MHz): 7.20 (5H, m); 4.95 (1H, m): 3.82 (1H, m); 3.48 (1H, m); 3.10 (2H, m); 2.87 (2H, m); 2.65 (1H, m); 2.34 (2H, m); 1.95 (4H, m); 1.70 (4H, m); 1.40 (2H, m).

## Beispiel 41

In analoger Weise wie in Beispiel 2 beschrieben, wurden aus 2.5 g Methyl 9(S)-[(äthoxy)(4-phenylbutyl)phosphinyl]-octahydro-6,10 -dioxo-6H-pyridazo[1,2-a][1,2]diazepin-1(S)-carboxylat 820 mg Methyl 9(S)-[(äthoxy)(4-phenylbutyl)phosphinyl]-octahydro-10-oxo -6H-pyridazo[1,2-a][1,2]diazepin-

-1(S)-carboxylat als leicht gelbes visköses Oel erhalten.

MS: m/e 450 ($M^+$).

## Beispiel 42

In analoger Weise wie in Beispiel 6 beschrieben, wurden aus 670 mg Methyl 9(S)-[(äthoxy)(4-phenylbutyl)phosphinyl]-octahydro-10-oxo -6H-pyridazo[1,2-a][1,2]diazepin-1(S)-carboxylat 270 mg des Dikaliumsalzes von 9(S)-[(Hydroxy)(4-phenylbutyl)phosphinyl] -octahydro-10-oxo-6H-pyridazo-[1,2-a][1,2]diazepin-1(S)-carbonsäure als weisses Lyophilisat erhalten.

NMR: δ($CD_3OD$, 400 MHz): 7.15 (5H, m); 4.70 (1H, m); 3.70 (1H, m); 3.60 (1H, m); 3.00 (2H, m); 2.60 (3H, m); 2.38 (1H, m); 1.97 (2H, m); 1.82 (2H, m); 1.60 (8H, m); 1.26 (1H, m).

## Beispiel 43

Ein Gemisch von 1.2 g 9-Methylen-octahydro-6,10-dioxo-6H -pyridazo[1,2-a][1,2]diazepin-1(S)-carbonsäure und 4.3 g Diäthyl 4-benzyloxybutylphosphonit in 25 ml Xylol wurde unter Stickstoff 24 Stunden auf 110°C erhitzt. Das Lösungsmittel wurde unter vermindertem Druck entfernt, und der Rückstand an Silicagel mit 5% Methanol in Aethylacetat als Eluierungsmittel chromatographiert. Dabei wurden 2,0 g Aethyl 9(S)-[[(4 -benzyloxybutyl)(äthoxy)phosphinyl]methyl]-octahydro-6,10 -dioxo-6H-pyridazo[1,2-a][1,2]diazepin-1(S)-carboxylat als visköses Oel erhalten.

MS: m/e 523 $(M+H)^+$.

Die als Ausgangsmaterial verwendete 9-Methylen-octahydro-6,10-dioxo-6H -pyridazo[1,2-a][1,2]diazepin-1(S)-carbonsäure wurde wie folgt hergestellt:

10 g tert.Butyl octahydro-9-methylen-6,10-dioxo-6H-pyridazo[1,2-a][1,2]diazepin -1(S)-carboxylat (hergestellt wie in Beispiel 48 beschrieben) wurden mit 40 ml Trifluoressigsäure 3 Stunden bei 20°C gerührt. Das Gemisch wurde eingedampft, und das erhaltene Oel mit Diäthyläther versetzt, wobei 7,6 g 9-Methylen-octahydro-6,10-dioxo-6H-pyridazo [1,2-a][1,2]diazepin-1(S)-carbonsäure als weisser Festkörper erhalten wurden, Schmelzpunkt 169°-172°C.

## Beispiel 44

In analoger Weise wie in Beispiel 6 beschrieben, wurden aus 700 mg Aethyl 9(S)-[[(4-benzyloxybutyl)(äthoxy)phosphinyl]methyl] -octahydro-6,10-dioxo-6H-pyridazo[1,2-a]-[1,2]diazepin -1(S)-carboxylat 400 mg 9(S)-[[(4-Benzyloxy-butyl)(hydroxy)phosphinyl]methyl] -octahydro-6,10-dioxo-6H-pyridazo[1,2-a][1,2]diazepin -1(S)-carbonsäure als farbloser Gummi erhalten.

NMR: δ($CD_3OD$, 300 MHz): 7.28 (5H, m); 5.32 (1H, dd, J = 3, 7Hz); 4.49 (2H, s); 4.47 (1H, m); 3.50 (3H, m); 3.12 (1H, m); 2.92 (1H, m); 2.35 (2H, m); 2.20 (2H, m); 1.90 (2H, m); 1.70 (9H, m).

## Beispiel 45

275 mg 9(S)-[[(4-Benzyloxybutyl)(hydroxy)phosphinyl]-methyl] -octahydro-6,10-dioxo-6H-pyridazo[1,2-a][1,2]diazepin -1(S)-carbonsäure wurden in 30 ml 3 ml Essigsäure enthaltendem Aethanol gelöst, und die Lösung über 10%-igem Palladium auf Kohle 16 Stunden hydriert. Der Katalysator wurde abfiltriert, und das Filtrat eingedampft. Der Rückstand wurde in Wasser aufgenommen, und der pH mit Kaliumbicarbonat auf 7 eingestellt. Eindampfen und Behandeln mit Aceton lieferte 190 mg des Dikaliumsalzes von 9(S)-[[(4-Hydroxybutyl)(hydroxy)phosphinyl]methyl]-octahydro-6,10-dioxo-6H-pyridazo[1,2-a][1,2]diazepin-1(S)-carbonsäure als

weissen hygroskopischen Festkörper.

NMR: δ($D_2O$, 300 MHz): 5.00 (1H, m); 4.35 (1H, m); 3.58 (3H, m); 3.08 (2H, m); 2.45 (1H, m); 2.22 (3H, m); 2.07 (2H, m); 1.93 (2H, m); 1.74-1.40 (7H, m).

## Beispiel 46

350 mg Aethyl 9(S)-[[(4-benzyloxybutyl)(äthoxy)phosphinyl]methyl] -octahydro-6,10-dioxo-6H-pyridazo[1,2-a]-[1,2]diazepin -1(S)-carboxylat wurden in einem Gemisch von 20 ml Aethanol und 2 ml Essigsäure gelöst, und die Lösung über 10%-igem Palladium auf Kohle 16 Stunden hydriert. Der Katalysator wurde abfiltriert, und das Filtrat eingedampft. Der Rückstand wurde in 5 ml Methylenchlorid aufgenommen und bei 20°C gerührt, während 0,18 ml Triäthylamin und danach eine Lösung von 0,075 ml Methansulfonylchlorid in 4 ml Methylenchlorid zugegeben wurden. Nach zweistündigem Weiterrühren wurde das Gemisch nacheinander mit 2N Salzsäure, Natriumbicarbonatlösung und gesättigter Natriumchloridlösung gewaschen. Die organischen Phasen wurden über wasserfreiem Magnesiumsulfat getrocknet und eingedampft. Der Rückstand wurde in 20 ml 2-Butanon gelöst, und dann wurden 60 mg Natriumazid zugegeben. Das Gemisch wurde 20 Stunden zum Rückfluss erhitzt und danach eingedampft. Der Rückstand wurde zwischen Aethylacetat und Wasser verteilt. Die organische Phase wurde eingedampft, und der Rückstand an Silicagel mit 10% Methanol in Aethylacetat als Eluierungsmittel chromatographiert, wobei 130 mg Aethyl 9(S)-[[(4-azidobutyl)(äthoxy)phosphinyl]methyl]-octahydro -6,10-dioxo-6H--pyridazo[1,2-a][1,2]diazepin-1(S) -carboxylat als Gummi erhalten wurden. Dieser Gummi wurde in 15 ml Aethanol aufgenommen, und die Lösung 3 Stunden über 10%-igem Palladium auf Kohle hydriert. Filtrieren und Eindampfen des Filtrats lieferte 120 mg Aethyl 9(S)-[[(4-aminobutyl)-(äthoxy)phosphinyl]methyl]-octahydro -6,10-dioxo-6H-pyri-

dazo[1,2-a][1,2]diazepin-1(S) -carboxylat als Gummi.

MS: m/e 431 ($M^+$).

## Beispiel 47

In analoger Weise wie in Beispiel 6 beschrieben, wurden aus 120 mg Aethyl 9(S)-[[(4-aminobutyl)- (äthoxy)phosphinyl]methyl]-octahydro -6,10-dioxo-6H-pyridazo[1,2-a]-[1,2]diazepin-1(S) -carboxylat 55 mg 9(S)-[[(4-Aminobutyl)-(hydroxy)phosphinyl]methyl]-octahydro -6,10-dioxo-6H-pyridazo[1,2-a][1,2]diazepin-1(S)-carbonsäure als leicht gelblicher Gummi erhalten.

| | |
|---|---|
| Analyse für | $C_{15}H_{26}N_3O_6P{\bullet}1.1\ H_2O$: |
| Berechnet | C, 45.59; H, 7.19; N, 10.63; $H_2O$ 5.01% |
| Gefunden | C, 45.72; H, 6.87; N, 10.54; $H_2O$ 5.12%. |

## Beispiel 48

Eine Lösung von 2.8 g 1-[2-(Methoxyphosphinyl)äthyl]-4--nitrobenzol und 3.6 g tert.Butyl octahydro-9-methylen-6,10--dioxo-6H -pyridazo[1,2-a][1,2]diazepin-1(S)-carboxylat in 80 ml Toluol wurde unter Stickstoff mit 2,49 g N,O-Bis(trimethylsilyl)acetamid versetzt. Nach 65 stündigem Erhitzen auf 60°C wurde die Lösung mit 100 ml Methylenchlorid verdünnt, mit Wasser gewaschen und eingedampft. Der Rückstand wurde an Silicagel mit 10% Methanol in Aethylacetat als Eluierungsmittel chromatographiert. Dabei wurde 1.0 g tert.Butyl octahydro-9(S)-[[methoxy(4 -nitrophenäthyl)phosphinyl]methyl]-6,10-dioxo-6H -pyridazo[1,2-a][1,2]diazepin-1(S)-carboxylat als leicht gelber Schaum erhalten.

NMR: δ($CDCl_3$, 300MHz): 8.16 (2H, d, J = 8Hz); 7.40 (2H, dd, J = 3, 8Hz); 5.30 (1H, m); 4.60 (1H, m); 3.72 (1.5H, d,

J = 9Hz); 3.68 (1.5H, d, J = 9Hz); 3.49 (1H, m); 3.05 (4H, m); 2.50 - 1.65 (11H, m); 1.48 (9H, s).

Das als Ausgangsmaterial verwendete tert.Butyl octahydro-9 -methylen-6,10-dioxo-6H-pyridazo[1,2-a][1,2]diazepin-1(S)-carboxylat wurde wie folgt hergestellt:

Eine Lösung von 10 g (0,0313 Mol) 1-Benzyloxycarbonyl-S--piperazinsäure-tert.butylester in 100 ml Methanol wurde bei Raumtemperatur und Atmosphärendruck über 5%-igem Palladium auf Kohle hydriert. Der Katalysator wurde abfiltriert, und das Filtrat zur Trockene eingedampft. Der zurückgebliebene rohe Piperazinsäure-t-butylester wurde in 100 ml Dioxan aufgenommen, und die auf 0°C abgekühlte Lösung mit einer Lösung von 3,94 g (0,0313 Mol) α-Methylenglutarsäureanhydrid in 100 ml Dioxan versetzt. Das Gemisch wurde 18 Stunden bei 20°C gerührt, und das Lösungsmittel durch Abdampfen entfernt. Der Rückstand wurde zwischen Methyl-tert.butyläther und gesättigter Natriumbiarbonatlösung verteilt. Die wässrige Phase wurde mit Salzsäure angesäuert und mit Methylenchlorid extrahiert, wobei man 8,34 g (85%) 3(S)-tert.-Butoxycarbonyl-hexahydro-α-methylen -δ-oxo-1-pyridazinpentansäure in Form von weissen Kristallen erhielt, Schmelzpunkt 96-99°C. 5,0 g (16 mMol) dieser Säure wurden in 350 ml Tetrahydrofuran aufgenommen, und die Lösung auf 0°C gekühlt. 3,75 g (18 mMol) Phosphorpentachlorid wurden zugegeben, und das Gemisch 1 Stunde bei 0°C und 18 Stunden bei 20°C gerührt. Das Lösungsmittel wurde abgedampft, und der Rückstand zwischen Aethylacetat und gesättigter Natriumbicarbonatlösung verteilt. Die organische Phase wurde eingedampft, und der Rückstand an Silicagel mit Aethylacetat/n-Hexan als Eluierungsmittel chromatographiert. Es wurden dabei 3,7 g (79%) tert.Butyl octahydro-9-methylen -6,10-dioxo-6H-pyridazo[1,2-a][1,2]diazepin -1(S)-carboxylat als weisser Festkörper erhalten, Schmelzpunkt 105-106°C (aus Hexan).

Das im ersten Absatz dieses Beispiels genannte 1-[2-(Methoxyphosphinyl)äthyl]-4-nitrobenzol wurde wie folgt hergestellt:

0.665 g kristalline Hypophosphorsäure wurde unter Stickstoff mit 1,73 g Trimethylorthoformat versetzt, und das Gemisch wurde 2 Stunden bei 20°C gerührt. Die erhaltene Lösung des rohen Methylhypophosphits wurde tropfenweise bei 0°C zu einer Lösung von 1 g 4-Nitrostyrol und 0,43 g Diisopropyläthylamin in 5 ml Methanol gegeben. Nach 65 Stunden bei 20°C wurde nocheinmal die gleiche Menge Methylhypophosphit zugegeben, und das Gemisch 24 Stunden bei 20°C weitergerührt. Das Gemisch wurde mit 30 ml Wasser verdünnt, und der pH mit wässriger Natriumbicarbonatlösung auf 7 eingestellt. Nach Extraktion mit Methylenchlorid und Chromatographie an Silicagel mit 10% Methanol in Aethylacetat als Eluierungsmittel wurde 0,5 g 1-[2-(Methoxyphosphinyl)äthyl]-4-nitrobenzol als gelbe Flüssigkeit erhalten.

## Beispiel 49

0.48 g tert.Butyl octahydro-9(S) -[[methoxy(4-nitrophenäthyl)phosphinyl]methyl]-6,10-dioxo -6H-pyridazo[1,2-a]-[1,2]diazepin-1(S)-carboxylat wurde in 2 ml Trifluoressigsäure 1 Stunde bei 20°C gerührt. Das Gemisch wurde eingedampft, und der Rückstand zwischen Natriumbicarbonatlösung und Methylenchlorid verteilt. Die wässrige Phase wurde mit 2N Salzsäure angesäuert und dann mit Methylenchlorid extrahiert. Eindampfen der Extrakte lieferte 0.29 g Octahydro-9(S)-[[methoxy(4-nitrophenäthyl)phosphinyl]methyl]-6,10-dioxo-6H-pyridazo[1,2-a][1,2]diazepin-1(S)-carbonsäure als weissen Schaum.

NMR: δ($CD_3OD$, 300 MHz): 8.19 (2H, dd, J = 8Hz); 7.53 (2H, dd, J = 3, 8Hz); 5.36 (1H, m); 4.51 (1H, m); 3.73

(1.5H, d, J = 9Hz); 3.69 (1.5H, d, J = 9Hz); 3.50 (1H, m); 3.05 (4H, m); 2.5 -- 1.6 (11H, m).

## Beispiel 50

Eine Lösung von 0.1 g Octahydro-9(S) -[[methoxy(4--nitrophenäthyl)phosphinyl]methyl] -6,10-dioxo-6H-pyridazo-[1,2-a][1,2]diazepin-1(S)-carbonsäure in 1 ml Acetonitril wurde unter Stickstoff mit 1 ml Trimethylsilylbromid versetzt. Die Lösung wurde 30 Minuten bei 20°C gerührt und dann eingedampft. Der Rückstand wurde in wässrigem Aceton gelöst, und die Lösung 30 Minuten gerührt und danach eingedampft. Der Rückstand wurde mit Diäthyläther versetzt, wobei 0.075 g Octahydro-9(S)-[[hydroxy(4 -nitrophenäthyl)phosphinyl]methyl]-6,10-dioxo-6H -pyridazo[1,2-a][1,2]diazepin-1(S)-carbonsäure als amorpher Festkörper erhalten wurde.

NMR: δ($CD_3OD$, 400 MHz); 8.19 (2H, d, J = 8Hz); 7.52 (2H, d, J = 8Hz); 5.35 (1H, m); 4.50 (1H,m); 3.52 (1H, m); 3.15 (1H, m); 2.98 (3H, m); 2.40 (2H, m); 2.24 (2H, m) 2.07 (2H, m); 1.90 (2H, m); 1.70 (3H, m).

## Beispiel 51

0.12 g Octahydro-9(S)-[[hydroxy(4-nitrophenäthyl)- phosphinyl]methyl]-6,10-dioxo-6H -pyridazo[1,2-a][1,2]diazepin--1(S)-carbonsäure in 50 ml Aethanol wurde über 10% Palladium auf Kohle 5 Stunden hydriert. Der Katalysator wurde abfiltriert, und das Filtrat eingedampft. Nach Behandeln das Rückstandes mit Diäthyläther wurde 0,1 g 9(S)-[[(4-Aminophenäthyl)- hydroxyphosphinyl]methyl] -octahydro-6,10-dioxo--6H-pyridazo[1,2-a][1,2]diazepin-1(S)-carbonsäure als amorpher Festkörper erhalten.

NMR: δ($D_2O$, 300 MHz): 7.43 (2H, d, J = 8Hz); 7.36 (2H, d, J = 8Hz); 5.23 (1H, m); 4.40 (1H, m); 3.39 (1H, m); 3.08

(2H, m); 2.90 (2H, m); 2.26 (4H, m); 1.91 (4H, m); 1.70 (2H, m); 1.58 (1H, m).

## Beispiel 52

In analoger Weise wie in Beispiel 51 beschrieben, wurde aus 0.125 g Octahydro-9(S)-[[methoxy(4-nitrophenäthyl)phosphinyl]methyl]-6,10-dioxo-6H -pyridazo[1,2-a][1,2]diazepin-1(S)-carbonsäure 0.112 g 9(S)-[[(4-Aminophenäthyl)methoxyphosphinyl]methyl] -octahydro-6,10-dioxo-6H-pyridazo[1,2-a]-[1,2]diazepin-1(S)-carbonsäure als Gummi erhalten.

MS: m/e 437 ($M^+$).

## Beispiel 53

0.11 g 9(S)-[[(4-Aminophenäthyl)methoxyphosphinyl]-methyl]-octahydro-6,10-dioxo-6H -pyridazo[1,2-a][1,2]diazepin-1(S)-carbonsäure wurde in 3 ml Acetonitril gelöst und mit 3 ml Trimethylsilylbromid versetzt. Die Lösung wurde 90 Stunden bei 20°C gerührt. Eindampfen und Behandeln des Rückstandes mit wässrigem Aceton gefolgt von Wiedereindampfen lieferte nach präparativer Hochdruckflüssigkeitschromatographie 0.025 g Octahydro 9(S)-[[(hydroxy)[4-(1 -iminoäthylamino)phenäthyl]phosphinyl]methyl]-6,10-dioxo -6H-pyridazo-[1,2-a][1,2]diazepin-1(S)-carbonsäure als amorphen Festkörper.

NMR: δ($CD_3OD$, 300MHz); 7.45 (2H, d, J = 8Hz); 7.26 (2H, d, J = 8Hz); 5.35 (1H, m); 4.50 (1H, m); 3.49 (1H, m); 3.15 (1H, m); 2.97 (3H, m); 2.39 (3H, s); 2.38 (2H, m); 2.24 (2H, m); 2.05 (2H, m); 1.89 (2H, m); 1.70 (3H, m).

Die folgenden Beispiele veranschaulichen pharmazeutische Präparate, enthaltend eine erfindungsgemässe Verbindung:

## Beispiel A

Tabletten, enthaltend die folgenden Bestandteile, können in herkömmlicher Weise hergesellt werden:

| Bestandteile | pro Tablette |
|---|---|
| Verbindung der Formel I | 10,0 mg |
| Lactose | 125,0 mg |
| Maisstärke | 75,0 mg |
| Talk | 4,0 mg |
| Magnesiumstearat | 1,0 mg |
| Tablettengewicht | 215,0 mg |

## Beispiel B

Kapseln, enthaltend die folgenden Bestandteile, können in herkömmlicher Weise hergestellt werden:

| Bestandteile | pro Kapsel |
|---|---|
| Verbindung der Formel I | 25,0 mg |
| Lactose | 150,0 mg |
| Maisstärke | 20,0 mg |
| Talk | 5,0 mg |
| Kapselfüllgewicht | 200,0 mg |

## Patentansprüche

1. Verbindungen der allgemeinen Formel

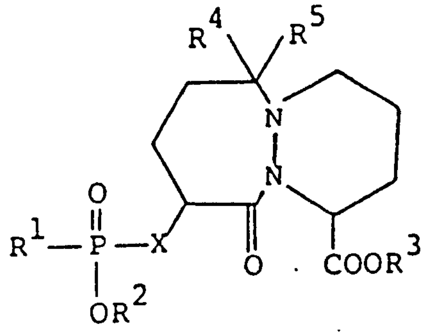

I

worin $R^1$ Hydroxy, Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy oder Aralkoxy, $R^2$ und $R^3$ je Wasserstoff, Alkyl oder Aralkyl, $R^4$ und $R^5$ je Wasserstoff oder zusammen eine Oxogruppe und X ein Sauerstoffatom oder die Gruppe $-NR^6-$ oder $-(CH_2)_n$ bedeuten, worin $R^6$ Wasserstoff, Alkyl oder Aralkyl und n 0, 1 oder 2 bedeuten,

und pharmazeutisch verwendbare Salze davon.

2. Verbindungen gemäss Anspruch 1, worin $R^1$ Hydroxy, Alkyl, Aralkyl oder Alkoxy bedeutet.

3. Verbindungen gemäss Anspruch 1 oder 2, worin $R^2$ Wasserstoff bedeutet.

4. Verbindungen gemäss einem der Ansprüche 1-3, worin $R^3$ Wasserstoff bedeutet.

5. Verbindungen gemäss einem der Ansprüche 1-4, worin X ein Sauerstoffatom oder die Gruppe -NH- oder $-(CH_2)_n-$ bedeutet, worin n 0 oder 1 bedeutet.

6. Verbindungen gemäss einem der Ansprüche 1-5, worin $R^1$ Hydroxy, Alkyl, Aralkyl oder Alkoxy, $R^2$ Wasserstoff, $R^3$ Wasserstoff und X ein Sauerstoffatom oder die Gruppe -NH- oder $-(CH_2)_n-$ bedeuten, worin n 0 oder 1 bedeutet.

7. Octahydro-9(S)-phosphonomethyl-10-oxo-6H-pyridazo-[1,2-a][1,2]diazepin-1(S)-carbonsäure.

8. Octahydro-9(S)-[[(hydroxy)methylphosphinyl]methyl]-6,10-dioxo-6H-pyridazo[1,2-a][1,2]diazepin-1(S)-carbonsäure.

9. Octahydro-9(S)-phosphono-6,10-dioxo-6H-pyridazo-[1,2-a][1,2]diazepin-1(S)-carbonsäure.

10. 9(S)-Diäthoxyphosphinyl-octahydro-6,10-dioxo-6H-pyridazo[1,2-a][1,2]diazepin-1(S)-carbonsäure.

11. Octahydro-9(S)-[[hydroxy(methoxy)phosphinyl]-methyl]-6,10-dioxo-6H-pyridazo[1,2-a][1,2]diazepin-1(S)-carbonsäure.

12. Octahydro-9(S)-phosphonomethyl-6,10-dioxo-6H-pyridazo[1,2-a][1,2]diazepin-1(S)-carbonsäure.

13. 9(S)-[[Hydroxy(3-phenylpropyl)phosphinyl)]methyl]-octahydro-6,10-dioxo-6H-pyridazo[1,2-a][1,2]diazepin-1(S)-carbonsäure.

14. 9(S)-[[Hydroxy(phenäthyl)phosphinyl]amino]-octahydro-10-oxo-6H-pyridazo[1,2-a][1,2]diazepin-1(S)-carbonsäure.

15. 9(S)-[[Hydroxy(2-phenäthyl)phosphinyl]methyl]-octahydro-10-oxo-6H-pyridazo[1,2-a][1,2]diazepin-1(S)-carbonsäure.

16. 9(S)-[Hydroxy(4-phenylbutyl)phosphinyl]-octahydro-10-oxo-6H-pyridazo[1,2-a][1,2]diazepin-1(S)-carbonsäure.

17.Methyl 9(S)-dimethoxyphosphinylmethyl-octahydro-6,10-dioxo-6H-pyridazo[1,2-a][1,2]diazepin-1(S)-carboxylat,

Methyl 9(S)-dimethoxyphosphinylmethyl-octahydro-10--oxo-6H-pyridazo[1,2-a][1,2]diazepin-1(S)-carboxylat,

Methyl octahydro-9(S)-phosphonomethyl-10-oxo-6H-pyridazo[1,2-a][1,2]diazepin-1(S)-carboxylat,

Methyl 9(S)-[[(äthoxy)methylphosphinyl]methyl]-octahydro-6,10-dioxo-6H-pyridazo[1,2-a][1,2]diazepin-1(S)-carboxylat,

Methyl 9(S)-diäthoxyphosphinyl-octahydro-6,10-dioxo--6H-pyridazo[1,2-a][1,2]diazepin-1(S)-carboxylat,

Methyl 9(S)-diäthoxyphosphinylmethyl-octahydro-6,10--dioxo-6H-pyridazo[1,2-a][1,2]diazepin-1(S)-carboxylat,

Methyl 9(S)-[[äthoxy(3-phenylpropyl)phosphinyl]-methyl]-octahydro-6,10-dioxo-6H-pyridazo[1,2-a][1,2]diazepine-1(S)-carboxylat,

Benzyl 9(S)-[[benzyloxy(phenäthyl)phosphinyl]amino]--octahydro-10-oxo-6H-pyridazo[1,2-a][1,2]diazepin-1(S)-carboxylat,

tert.Butyl 9(S)-(dibenzylphosphonoxy)-octahydro-6,10--dioxo-6H-pyridazo[1,2-a][1,2]diazepin-1(S)-carboxylat,

tert.Butyl 9(S)-[benzyloxy(phenäthyl)phosphinyloxy]--octahydro-6,10-dioxo-6H-pyridazo[1,2-a][1,2]diazepin-1(S)-carboxylat,

Benzyl 9(S)-[(dibenzyloxyphosphinyl)amino]-octahydro--10-oxo-6H-pyridazo[1,2-a][1,2]diazepin-1(S)-carboxylat und

9(S)-[Dibenzyloxyphosphinyl)amino]-octahydro-10-oxo-6H-pyridazo[1,2-a][1,2]diazepin-1(S)-carbonsäure.

18. tert.Butyl 9(S)-[benzyloxy(4-phenylbutyl)phosphinyloxy]-octahydro-6,10-dioxo-6H-pyridazo[1,2-a][1,2]diazepin-1(S)-carboxylat,

9(S)-[Hydroxy(4-phenylbutyl)phosphinyloxy]-octahydro-6,10-dioxo-6H-pyridazo[1,2-a][1,2]diazepin-1(S)-carbonsäure,

Benzyl 9(S)-[[benzyloxy(benzyl)phosphinyl]amino]-octahydro-10-oxo-6H-pyridazo[1,2-a][1,2]diazepin-1(S)-carboxylat,

Benzyl 9(S)-[[benzyloxy(3-phenylpropyl)phosphinyl]-amino]-octahydro-10-oxo-6H-pyridazo[1,2-a][1,2]diazepin-1(S)-carboxylat,

Benzyl 9(S)-[[benzyloxy(4-phenylbutyl)phosphinyl]-amino]-octahydro-10-oxo-6H-pyridazo[1,2-a][1,2]diazepin-1(S)-carboxylat,

9(S)-[[Hydroxy(benzyl)phosphinyl]amino]-octahydro-10-oxo-6H-pyridazo[1,2-a][1,2]diazepin-1(S)-carbonsäure,

9(S)-[[Hydroxy(3-phenylpropyl)phosphinyl]amino]-octahydro-10-oxo-6H-pyridazo[1,2-a][1,2]diazepin-1(S)-carbonsäure,

9(S)-[[Hydroxy(4-phenylbutyl)phosphinyl]amino]-octahydro-10-oxo-6H-pyridazo[1,2-a][1,2]diazepin-1(S)-carbonsäure,

Methyl 9(S)-[(äthoxy)(5-phenylpentyl)phosphinyl]-octahydro-6,10-dioxo-6H-pyridazo[1,2-a][1,2]diazepin-1(S)-carboxylat,

Methyl 9(S)-[(äthoxy)(4-phenylbutyl)phosphinyl]-octahydro-6,10-dioxo-6H-pyridazo[1,2-a][1,2]diazepin-1(S)-carboxylat,

Methyl 9(S)-[(äthoxy)(3-phenylpropyl)phosphinyl]-octahydro-6,10-dioxo-6H-pyridazo[1,2-a][1,2]diazepin-1(S)-carboxylat,

Methyl 9(S)-[(äthoxy)(phenäthyl)phosphinyl]-octahydro-6,10-dioxo-6H-pyridazo[1,2-a][1,2]diazepin-1(S)-carboxylate,

9(S)-[Hydroxy(5-phenylpentyl)phosphinyl]-octahydro-6,10-dioxo-6H-pyridazo[1,2-a][1,2]diazepin-1(S)-carbonsäure,

9(S)-[Hydroxy(4-phenylbutyl)phosphinyl]-octahydro-6,10--dioxo-6H-pyridazo[1,2-a][1,2]diazepin-1(S)-carbonsäure,

9(S)-[Hydroxy(3-phenylpropyl)phosphinyl]-octahydro-6,10-dioxo-6H-pyridazo[1,2-a][1,2]diazepin-1(S)-carbonsäure,

9(S)-[Hydroxy(phenäthyl)phosphinyl]octahydro-6,10--dioxo-6H-pyridazo[1,2-a][1,2]diazepin-1(S)-carbonsäure,

Methyl 9(S)-[[äthoxy(phenäthyl)phosphinyl]methyl]-octahydro-6,10-dioxo-6H-pyridazo[1,2-a][1,2]diazepin-1(S)--carboxylat,

9(S)-[[Hydroxy(phenäthyl)phosphinyl]methyl]octahydro--6,10-dioxo-6H-pyridazo[1,2-a][1,2]diazepin-1(S)-carbonsäure,

Methyl 9(S)-[[äthoxy(phenäthyl)phosphinyl]methyl]-octahydro-10-oxo-6H-pyridazo[1,2-a][1,2]diazepin-1(S)--carboxylat,

Methyl 9(S)-[(äthoxy)(4-phenylbutyl)phosphinyl]-octahydro-10-oxo-6H-pyridazo[1,2-a][1,2]diazepin-1(S)-carboxylat,

Aethyl 9(S)-[[(4-benzyloxybutyl)(äthoxy)phosphinyl]-methyl]-octahydro-6,10-dioxo-6H-pyridazo[1,2-a][1,2]diazepin-1(S)-carboxylat,

9(S)-[[(4-Benzyloxybutyl)(hydroxy)phosphinyl]methyl]--octahydro-6,10-dioxo-6H-pyridazo[1,2-a][1,2]diazepin-1(S)-carbonsäure,

9(S)-[[(4-Hydroxybutyl)(hydroxy)phosphinyl]methyl]-octahydro-6,10-dioxo-6H-pyridazo[1,2-a][1,2]diazepin-1(S)--carbonsäure,

9(S)-[[(4-Aminobutyl)(äthoxy)phosphinyl]methyl]-octahydro-6,10-dioxo-6H-pyridazo[1,2-a][1,2]diazepin-1(S)-carboxylat,

9(S)-[[(4-Aminobutyl)(hydroxy)phosphinyl]methyl]-octahydro-6,10-dioxo-6H-pyridazo[1,2-a][1,2]diazepin-1(S)-carbonsäure,

tert.Butyl octahydro-9(S)-[[methoxy(4-nitrophenäthyl)-phosphinyl]methyl]-6,10-dioxo-6H-pyridazo[1,2-a][1,2]diazepin-1(S)-carboxylat,

Octahydro-9(S)-[[methoxy(4-nitrophenäthyl)phosphinyl]-methyl]-6,10-dioxo-6H-pyridazo[1,2-a][1,2]diazepin-1(S)--carbonsäure,

Octahydro-9(S)-[[hydroxy(4-nitrophenäthyl)phosphinyl]-methyl]-6,10-dioxo-6H -pyridazo[1,2-a][1,2]diazepin-1(S)--carbonsäure,

9(S)-[[(4-Aminophenäthyl)hydroxyphosphinyl]methyl]-octahydro-6,10-dioxo-6H-pyridazo[1,2-a][1,2]diazepin-1(S)-carbonsäure,

9(S)-[[(4-Aminophenäthyl)methoxyphosphinyl]methyl]-octahydro-6,10-dioxo-6H-pyridazo[1,2-a][1,2]diazepin-1(S)-carbonsäure und

Octahydro 9(S)-[[(hydroxy)[4-(1-iminoäthylamino)phenäthyl]phosphinyl]methyl]-6,10-dioxo-6H-pyridazo[1,2-a]-[1,2]diazepin-1(S)-carbonsäure.

19. Verbindungen der allgemeinen Formel

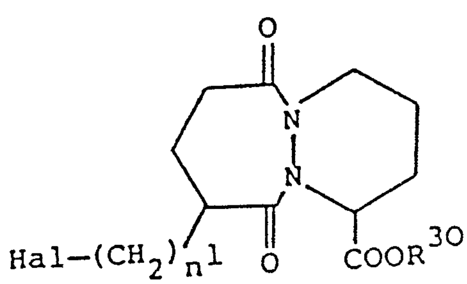

IIa

worin $R^{30}$ Alkyl oder Aralkyl, Hal ein Halogenatom und $n^1$ 1 oder 2 bedeuten.

20. Verbindungen der allgemeinen Formel

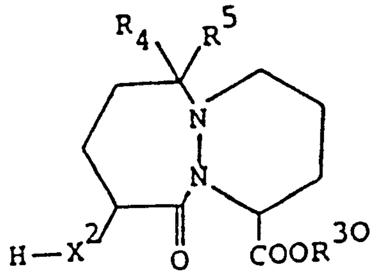

IVa

worin $R^4$ und $R^5$ je Wasserstoff oder zusammen eine Oxogruppe, $R^{30}$ Alkyl oder Aralkyl und $X^2$ ein Sauerstoffatom oder die Gruppe $-NR^{61}-$ bedeuten, worin $R^{61}$ Alkyl oder Aralkyl bedeutet.

21. Verbindung gemäss einem der Ansprüche 1-18 zur Anwendung als therapeutischer Wirkstoff.

22. Verbinung gemäss einem der Ansprüche 1-18 zur Anwendung als Antihypertensivum.

23. Verfahren zur Herstellung einer Verbindung gemäss einem der Ansprüche 1-18, dadurch gekennzeichnet, dass man

a) zur Herstellung einer Verbindung der Formel I, worin $R^1$ Alkyl, Aralkyl, Alkoxyalkyl, Acylaminoalkyl, Dialkylaminoalkyl, Alkoxycarbonylaminoalkyl, Alkoxycarbonylalkyl, Alkoxy oder Aralkoxy, $R^2$ und $R^3$ je Alkyl oder Aralkyl, $R^4$ und $R^5$ zusammen eine Oxogruppe und X $-(CH_2)_n-$ bedeuten, worin n die oben angegebene Bedeutung besitzt, eine Verbindung der allgemeinen Formel

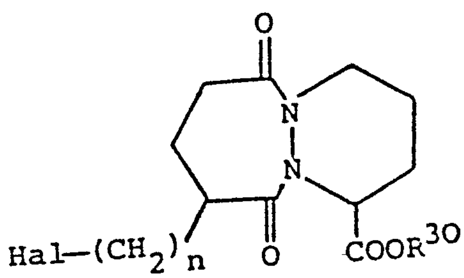

II

worin n die oben angegebenen Bedeutungen besitzt und $R^{30}$ Alkyl oder Aralkyl und Hal ein Halogenatom bedeuten,

mit einer Verbindung der allgemeinen Formel

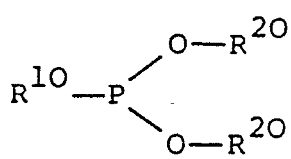

III

worin $R^{10}$ Alkyl, Aralkyl, Aralkoxyalkyl, Acylaminoalkyl, Dialkylaminoalkyl, Alkoxycarbonylaminoalkyl, Alkoxycarbonylalkyl, Alkoxy oder Aralkoxy und $R^{20}$ Alkyl oder Aralkyl bedeuten,

umsetzt,

oder

b) zur Herstellung einer Verbindung der Formel I, worin $R^1$ Alkyl, Aralkyl, Aralkoxyalkyl, Acylaminoalkyl, Dialkylaminoalkyl, Alkoxycarbonylaminoalkyl, Alkoxycarbonylalkyl, Alkoxy oder Aralkoxy, $R^2$ und $R^3$ je Alkyl oder Aralkyl und X ein Sauerstoffatom oder die Gruppe $-NR^6-$ bedeuten, worin $R^6$ Wasserstoff, Alkyl oder

Aralkyl bedeutet, eine Verbindung der allgemeinen Formel

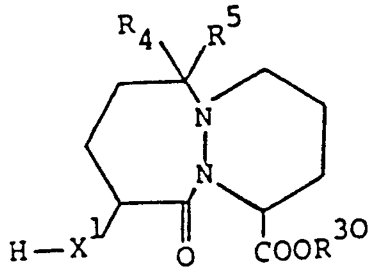

IV

worin $R^4$, $R^5$ und $R^{30}$ die oben angegebene Bedeutung besitzen und $X^1$ ein Sauerstoffatom oder die Gruppe $-NR^6-$ bedeutet, worin $R^6$ die oben angegebene Bedeutung besitzt,

mit einer Verbindung der allgemeinen Formel

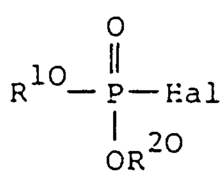

V

worin $R^{10}$, $R^{20}$ und Hal die oben angegebene Bedeutung besitzen,

umsetzt,

oder

c) zur Herstellung einer Verbindung der Formel I, worin $R^2$ Alkyl oder Aralkyl, $R^4$ und $R^5$ zusammen eine Oxogruppe und X $-CH_2-$ bedeuten, eine Verbindung der allgemeinen Formel

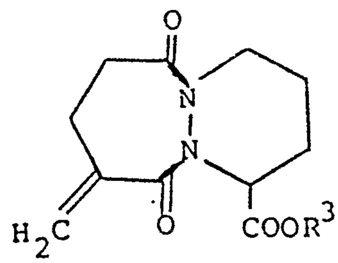

VI

worin $R^3$ die oben angegebene Bedeutung besitzt, mit einer Verbindung der allgemeinen Formel

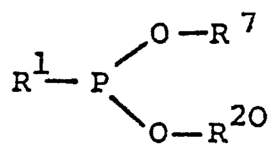

VII

worin $R^1$ und $R^{20}$ die oben angegebene Bedeutung besitzen und $R^7$ Alkyl, Aralkyl oder Trialkylsilyl bedeutet,

umsetzt,

oder

d) zur Herstellung einer Verbindung der Formel I, worin $R^1$ Alkyl, Aralkyl, Aralkoxyalkyl, Aminoalkyl, Acylaminoalkyl, Monoalkylaminoalkyl, Dialkylaminoalkyl, Alkoxycarbonylaminoalkyl, Alkoxycarbonylalkyl, Alkoxy oder Aralkoxy, $R^2$ und $R^3$ je Alkyl oder Aralkyl und $R^4$ und $R^5$ je Wasserstoff bedeuten, eine Verbindung der Formel I, worin $R^1$ Alkyl, Aralkyl, Aralkoxyalkyl, Aminoalkyl, Acylaminoalkyl, Monoalkylaminoalkyl, Dialkylaminoalkyl, Alkoxycarbonylaminoalkyl, Alkoxycarbonylalkyl, Alkoxy oder

Aralkoxy, $R^2$ und $R^3$ je Alkyl oder Aralkyl und $R^4$ und $R^5$ zusammen eine Oxogruppe bedeuten, reduziert, oder

e) zur Herstellung einer Verbindung der Formel I, worin $R^1$ Hydroxy, Alkoxy oder Aralkoxy, $R^2$ Wasserstoff, Alkyl oder Aralkyl und/oder $R^3$ Wasserstoff, Alkyl oder Aralkyl bedeuten, mit der Auflage, dass entweder $R^1$ Hydroxy und/oder mindestens einer von $R^2$ und $R^3$ Wasserstoff bedeutet, in einer Verbindung der Formel I, worin $R^1$ Hydroxy, Alkoxy oder Aralkoxy, $R^2$ Wasserstoff, Alkyl oder Aralkyl und/oder $R^3$ Wasserstoff, Alkyl oder Aralkyl bedeuten, mit der Massgabe, dass entweder $R^1$ von Hydroxy und/oder mindestens einer von $R^2$ und $R^3$ von Wasserstoff verschieden ist, die Estergruppe abspaltet, oder

f) zur Herstellung einer Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy oder Aralkoxy, $R^2$ und $R^3$ je Alkyl oder Aralkyl und X die Gruppe $-NR^{61}-$ bedeuten, worin $R^{61}$ Alkyl oder Aralkyl bedeutet, eine Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy oder Aralkoxy, $R^2$ und $R^3$ je Alkyl oder Aralkyl und X die Gruppe -NH- bedeuten, alkyliert oder aralkyliert, oder

g) zur Herstellung einer Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkyl oder Aralkyl, $R^2$ Alkyl oder Aralkyl und $R^3$ Alkyl oder Aralkyl bedeuten, eine Verbindung der Formel I, worin $R^1$ Alkyl, Aralkyl, Aralkoxy-

alkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkyl oder Aralkyl, $R^2$ Alkyl oder Aralkyl und $R^3$ Wasserstoff bedeuten, verestert, oder

h) zur Herstellung einer Verbindung der Formel I, worin $R^1$ Hydroxyalkyl und $R^2$ und $R^3$ je Wasserstoff oder Alkyl bedeuten, eine Verbindung der Formel I, worin $R^1$ Aryloxyalkyl und $R^2$ und $R^3$ je Wasserstoff oder Alkyl bedeuten, katalytisch hydriert, oder

i) zur Herstellung einer Verbindung der Formel I, worin $R^1$ Haloalkyl und $R^2$ und $R^3$ je Alkyl oder Aralkyl bedeuten, eine Verbindung der Formel I, worin $R^1$ Hydroxyalkyl und $R^2$ und $R^3$ je Alkyl oder Aralkyl bedeuten, halogeniert, oder

j) zur Herstellung einer Verbindung der Formel I, worin $R^1$ Aminoalkyl oder Monoalkylaminoalkyl und $R^2$ und $R^3$ je Alkyl oder Aralkyl bedeuten, die Hydroxyalkylgruppe in einer Verbindung der Formel I, worin $R^1$ Hydroxyalkyl und $R^2$ und $R^3$ je Alkyl oder Aralkyl bedeuten, in eine Aminoalkyl- oder Monoalkylaminoalkylgruppe überführt, oder

k) zur Herstellung einer Verbindung der Formel I, worin $R^1$ Aminoalkyl oder Monoalkylaminoalkyl und $R^2$ und $R^3$ je Alkyl oder Aralkyl bedeuten, eine Verbindung der Formel I, worin $R^1$ Haloalkyl und $R^2$ und $R^3$ je Alkyl oder Aralkyl bedeuten, mit Ammoniak oder einem Monoalkylamin umsetzt, oder

l) zur Herstellung einer Verbindung der Formel I, worin $R^1$ Acylaminoalkyl und $R^2$ und $R^3$ je Alkyl oder Aralkyl bedeuten, eine Verbindung der Formel I, worin $R^1$ Aminoalkyl und $R^2$ und $R^3$ je Alkyl oder Aralkyl

bedeuten, acyliert, oder

m) zur Herstellung einer Verbindung der Formel I, worin $R^1$ Carboxyalkyl und $R^2$ und $R^3$ je Alkyl oder Aralkyl bedeuten, eine Verbindung der Formel I, worin $R^1$ Hydroxyalkyl und $R^2$ und $R^3$ je Alkyl oder Aralkyl bedeuten, oxydiert, und

n) erwünschtenfalls eine im Substituenten $R^1$ in einer Verbindung der Formel I vorhandene nitrosubstituierte Phenylgruppe zur aminosubstituierten Phenylgruppe reduziert, und/oder

o) erwünschtenfalls eine im Substituenten $R^1$ in einer Verbindung der Formel I vorhandene aminosubstituierte Phenylgruppe durch Umsetzen mit einem Alkylcyanid in Gegenwart eines Trialkylsilylhalogenids in eine Iminoalkylaminogruppe überführt, und/oder

p) erwünschtenfalls ein erhaltenes Gemisch von diastereoisomeren Racematen in die entsprechenden diastereoisomeren Racemate oder optisch reinen Diastereoisomeren auftrennt, und/oder

q) erwünschtenfalls ein erhaltenes Racemat in die beiden optischen Antipoden auftrennt, und

r) erwünschtenfalls eine erhaltene Verbindung der Formel I in ein pharmazeutisch verwendbares Salz überführt.

24. Verfahren gemäss Anspruch 23, dadurch gekennzeichnet, dass man die Verbindungen der Formel I und deren pharmazeutisch verwendbare Salze nach den Verfahrensvarianten a), b), d), e), f), g), h), i), j), k), l), m), p), q) und/oder r) herstellt.

25. Arzneimittel, enthaltend eine Verbindung gemäss einem der Ansprüche 1-18 und ein therapeutisch inertes Excipiens.

26. Antihypertensivum, enthaltend eine Verbindung gemäss einem der Ansprüche 1-18 und ein therapeutisch inertes Excipiens.

27. Verwendung einer Verbindung gemäss einem der Ansprüche 1-18 zur Herstellung eines Antihypertensivums.

***

Patentansprüche für den Vertragsstaat AT

1. Verfahren zur Herstellung von Verbindungen der allgemeinen Formel

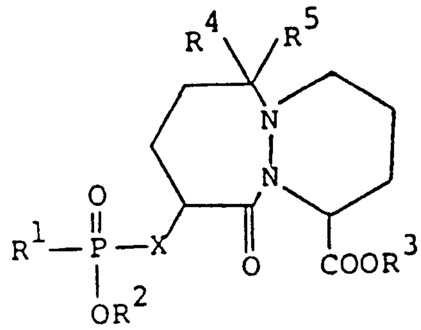

I

worin $R^1$ Hydroxy, Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy oder Aralkoxy, $R^2$ und $R^3$ je Wasserstoff, Alkyl oder Aralkyl, $R^4$ und $R^5$ je Wasserstoff oder zusammen eine Oxogruppe und X ein Sauerstoffatom oder die Gruppe $-NR^6-$ oder $-(CH_2)_n-$ bedeuten, worin $R^6$ Wasserstoff, Alkyl oder Aralkyl und n 0, 1 oder 2 bedeuten,

und pharmazeutisch verwendbare Salze davon, dadurch gekennzeichnet, dass man

a) zur Herstellung einer Verbindung der Formel I, worin $R^1$ Alkyl, Aralkyl, Alkoxyalkyl, Acylaminoalkyl, Dialkylaminoalkyl, Alkoxycarbonylaminoalkyl, Alkoxycarbonylalkyl, Alkoxy oder Aralkoxy, $R^2$ und $R^3$ je Alkyl oder Aralkyl, $R^4$ und $R^5$ zusammen eine Oxogruppe und X $-(CH_2)_n-$ bedeuten, worin n die oben angegebene Bedeutung besitzt, eine Verbindung der allgemeinen Formel

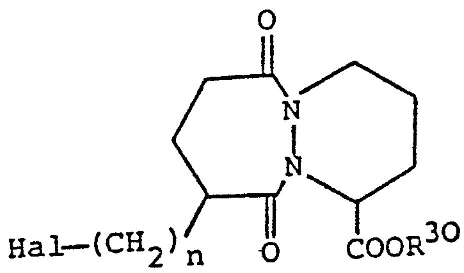

II

worin n die oben angegebenen Bedeutungen besitzt und $R^{30}$ Alkyl oder Aralkyl und Hal ein Halogenatom bedeuten,

mit einer Verbindung der allgemeinen Formel

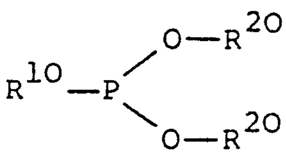

III

worin $R^{10}$ Alkyl, Aralkyl, Aralkoxyalkyl, Acylaminoalkyl, Dialkylaminoalkyl, Alkoxycarbonylaminoalkyl, Alkoxycarbonylalkyl, Alkoxy oder Aralkoxy und $R^{20}$ Alkyl oder Aralkyl bedeuten,

umsetzt,

oder

b) zur Herstellung einer Verbindung der Formel I, worin $R^1$ Alkyl, Aralkyl, Aralkoxyalkyl, Acylaminoalkyl, Dialkylaminoalkyl, Alkoxycarbonylaminoalkyl, Alkoxycarbonylalkyl, Alkoxy oder Aralkoxy, $R^2$ und $R^3$ je Alkyl oder Aralkyl und X ein Sauerstoffatom oder die Gruppe $-NR^6-$ bedeuten, worin $R^6$ Wasserstoff, Alkyl oder

Aralkyl bedeutet, eine Verbindung der allgemeinen Formel

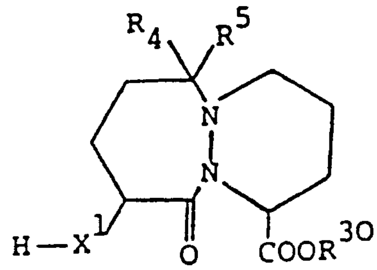

IV

worin $R^4$, $R^5$ und $R^{30}$ die oben angegebene Bedeutung besitzen und $X^1$ ein Sauerstoffatom oder die Gruppe $-NR^6-$ bedeutet, worin $R^6$ die oben angegebene Bedeutung besitzt,

mit einer Verbindung der allgemeinen Formel

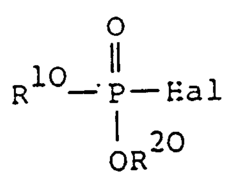

V

worin $R^{10}$, $R^{20}$ und Hal die oben angegebene Bedeutung besitzen,

umsetzt,

oder

c) zur Herstellung einer Verbindung der Formel I, worin $R^2$ Alkyl oder Aralkyl, $R^4$ und $R^5$ zusammen eine Oxogruppe und X $-CH_2-$ bedeuten, eine Verbindung der allgemeinen Formel

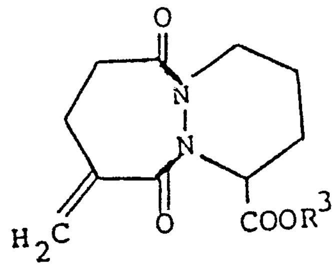

VI

worin $R^3$ die oben angegebene Bedeutung besitzt, mit einer Verbindung der allgemeinen Formel

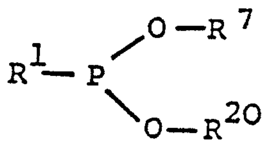

VII

worin $R^1$ und $R^{20}$ die oben angegebene Bedeutung besitzen und $R^7$ Alkyl, Aralkyl oder Trialkylsilyl bedeutet,

umsetzt.

oder

d) zur Herstellung einer Verbindung der Formel I, worin $R^1$ Alkyl, Aralkyl, Aralkoxyalkyl, Aminoalkyl, Acylaminoalkyl, Monoalkylaminoalkyl, Dialkylaminoalkyl, Alkoxycarbonylaminoalkyl, Alkoxycarbonylalkyl, Alkoxy oder Aralkoxy, $R^2$ und $R^3$ je Alkyl oder Aralkyl und $R^4$ und $R^5$ je Wasserstoff bedeuten, eine Verbindung der Formel I, worin $R^1$ Alkyl, Aralkyl, Aralkoxyalkyl, Aminoalkyl, Acylaminoalkyl, Monoalkylaminoalkyl, Dialkylaminoalkyl, Alkoxycarbonylaminoalkyl, Alkoxycarbonylalkyl, Alkoxy oder

Aralkoxy, $R^2$ und $R^3$ je Alkyl oder Aralkyl und $R^4$ und $R^5$ zusammen eine Oxogruppe bedeuten, reduziert, oder

e) zur Herstellung einer Verbindung der Formel I, worin $R^1$ Hydroxy, Alkoxy oder Aralkoxy, $R^2$ Wasserstoff, Alkyl oder Aralkyl und/oder $R^3$ Wasserstoff, Alkyl oder Aralkyl bedeuten, mit der Auflage, dass entweder $R^1$ Hydroxy und/oder mindestens einer von $R^2$ und $R^3$ Wasserstoff bedeutet, in einer Verbindung der Formel I, worin $R^1$ Hydroxy, Alkoxy oder Aralkoxy, $R^2$ Wasserstoff, Alkyl oder Aralkyl und/oder $R^3$ Wasserstoff, Alkyl oder Aralkyl bedeuten, mit der Massgabe, dass entweder $R^1$ von Hydroxy und/oder mindestens einer von $R^2$ und $R^3$ von Wasserstoff verschieden ist, die Estergruppe abspaltet, oder

f) zur Herstellung einer Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy oder Aralkoxy, $R^2$ und $R^3$ je Alkyl oder Aralkyl und X die Gruppe $-NR^{61}-$ bedeuten, worin $R^{61}$ Alkyl oder Aralkyl bedeutet, eine Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkoxy oder Aralkoxy, $R^2$ und $R^3$ je Alkyl oder Aralkyl und X die Gruppe -NH- bedeuten, alkyliert oder aralkyliert, oder

g) zur Herstellung einer Verbindung der Formel I, worin $R^1$ Alkyl, Aralkyl, Aralkoxyalkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkyl oder Aralkyl, $R^2$ Alkyl oder Aralkyl und $R^3$ Alkyl oder Aralkyl bedeuten, eine Verbindung der Formel I, worin $R^1$ Alkyl, Aralkyl, Aralkoxy-

alkyl, Hydroxyalkyl, Aminoalkyl, Acylaminoalkyl, Monoalkylaminoalkyl, Dialkylaminoalkyl, Alkoxycarbonylaminoalkyl, Haloalkyl, Carboxyalkyl, Alkoxycarbonylalkyl, Alkyl oder Aralkyl, $R^2$ Alkyl oder Aralkyl und $R^3$ Wasserstoff bedeuten, verestert, oder

h) zur Herstellung einer Verbindung der Formel I, worin $R^1$ Hydroxyalkyl und $R^2$ und $R^3$ je Wasserstoff oder Alkyl bedeuten, eine Verbindung der Formel I, worin $R^1$ Aryloxyalkyl und $R^2$ und $R^3$ je Wasserstoff oder Alkyl bedeuten, katalytisch hydriert, oder

i) zur Herstellung einer Verbindung der Formel I, worin $R^1$ Haloalkyl und $R^2$ und $R^3$ je Alkyl oder Aralkyl bedeuten, eine Verbindung der Formel I, worin $R^1$ Hydroxyalkyl und $R^2$ und $R^3$ je Alkyl oder Aralkyl bedeuten, halogeniert, oder

j) zur Herstellung einer Verbindung der Formel I, worin $R^1$ Aminoalkyl oder Monoalkylaminoalkyl und $R^2$ und $R^3$ je Alkyl oder Aralkyl bedeuten, die Hydroxyalkylgruppe in einer Verbindung der Formel I, worin $R^1$ Hydroxyalkyl und $R^2$ und $R^3$ je Alkyl oder Aralkyl bedeuten, in eine Aminoalkyl- oder Monoalkylaminoalkylgruppe überführt, oder

k) zur Herstellung einer Verbindung der Formel I, worin $R^1$ Aminoalkyl oder Monoalkylaminoalkyl und $R^2$ und $R^3$ je Alkyl oder Aralkyl bedeuten, eine Verbindung der Formel I, worin $R^1$ Haloalkyl und $R^2$ und $R^3$ je Alkyl oder Aralkyl bedeuten, mit Ammoniak oder einem Monoalkylamin umsetzt, oder

l) zur Herstellung einer Verbindung der Formel I, worin $R^1$ Acylaminoalkyl und $R^2$ und $R^3$ je Alkyl oder Aralkyl bedeuten, eine Verbindung der Formel I, worin $R^1$ Aminoalkyl und $R^2$ und $R^3$ je Alkyl oder Aralkyl

bedeuten, acyliert, oder

m) zur Herstellung einer Verbindung der Formel I, worin $R^1$ Carboxyalkyl und $R^2$ und $R^3$ je Alkyl oder Aralkyl bedeuten, eine Verbindung der Formel I, worin $R^1$ Hydroxyalkyl und $R^2$ und $R^3$ je Alkyl oder Aralkyl bedeuten, oxydiert, und

n) erwünschtenfalls eine im Substituenten $R^1$ in einer Verbindung der Formel I vorhandene nitrosubstituierte Phenylgruppe zur aminosubstituierten Phenylgruppe reduziert, und/oder

o) erwünschtenfalls eine im Substituenten $R^1$ in einer Verbindung der Formel I vorhandene aminosubstituierte Phenylgruppe durch Umsetzen mit einem Alkylcyanid in Gegenwart eines Trialkylsilylhalogenids in eine Iminoalkylaminogruppe überführt, und/oder

p) erwünschtenfalls ein erhaltenes Gemisch von diastereoisomeren Racematen in die entsprechenden diastereoisomeren Racemate oder optisch reinen Diastereoisomeren auftrennt, und/oder

q) erwünschtenfalls ein erhaltenes Racemat in die beiden optischen Antipoden auftrennt, und

r) erwünschtenfalls eine erhaltene Verbindung der Formel I in ein pharmazeutisch verwendbares Salz überführt.

2. Verfahren gemäss Anspruch 1, dadurch gekennzeichnet, dass man die Verbindungen der Formel I und deren pharmazeutisch verwendbare Salze nach den Verfahrensvarianten a), b), d), e), f), g), h), i), j), k), l), m), p), q) und/oder r) herstellt.

3. Verfahren gemäss Anspruch 1 oder 2, worin $R^1$ Hydroxy, Alkyl, Aralkyl oder Alkoxy bedeutet.

4. Verfahren gemäss einem der Ansprüche 1-3, worin $R^2$ Wasserstoff bedeutet.

5. Verfahren gemäss einem der Ansprüche 1-4, worin $R^3$ Wasserstoff bedeutet.

6. Verfahren gemäss einem der Ansprüche 1-5, worin X ein Sauerstoffatom oder die Gruppe -NH- oder $-(CH_2)_n-$ bedeutet, worin n 0 oder 1 bedeutet.

7. Verfahren gemäss einem der Ansprüche 1-6, worin $R^1$ Hydroxy, Alkyl, Aralkyl oder Alkoxy, $R^2$ Wasserstoff, $R^3$ Wasserstoff und X ein Sauerstoffatom oder die Gruppe -NH- oder $-(CH_2)_n$ bedeuten, worin n 0 oder 1 bedeutet.

8. Verfahren gemäss Anspruch 2, dadurch gekennzeichnet, dass man Octahydro-9(S)-phosphonomethyl-10-oxo-6H-pyridazo[1,2-a][1,2]-diazepin-1(S)-carbonsäure herstellt.

9. Verfahren gemäss Anspruch 2, dadurch gekennzeichnet, dass man Octahydro-9(S)-[[(hydroxy)methylphosphinyl]-methyl]-6,10 -dioxo-6H-pyridazo[1,2-a][1,2]diazepin-1(S)-carbonsäure herstellt.

10. Verfahren gemäss Anspruch 2, dadurch gekennzeichnet, dass man Octahydro-9(S)-phosphono-6,10-dioxo-6H-pyridazo[1,2-a][1,2]diazepin-1(S)-carbonsäure herstellt.

11. Verfahren gemäss Anspruch 2, dadurch gekennzeichnet, dass man 9(S)-Diäthoxyphosphinyl-octahydro-6,10-dioxo-6H -pyridazo[1,2-a][1,2]diazepin-1(S)-carbonsäure herstellt.

12. Verfahren gemäss Anspruch 2, dadurch gekennzeichnet, dass man Octahydro-9(S)-[[hydroxy(methoxy)phosphinyl]methyl]-6,10 -dioxo-6H-pyridazo[1,2-a][1,2]diazepin-1(S)-carbonsäure herstellt.

13. Verfahren gemäss Anspruch 2, dadurch gekennzeichnet, dass man Octahydro-9(S)-phosphonomethyl-6,10-dioxo-6H -pyridazo[1,2-a][1,2]diazepin-1(S)-carbonsäure herstellt.

14. Verfahren gemäss Anspruch 2, dadurch gekennzeichnet, dass man 9(S)-[[Hydroxy-(3-phenylpropyl)phosphinyl)]-methyl] -octahydro-6,10-dioxo-6H-pyridazo[1,2-a][1,2]diazepin-1(S) -carbonsäure herstellt.

15. Verfahren gemäss Anspruch 2, dadurch gekennzeichnet, dass man 9(S)-[[Hydroxy(phenäthyl)phosphinyl]amino]-octahydro-10-oxo -6H-pydridazo[1,2-a][1,2]diazepin-1(S)-carbonsäure herstellt.

16. Verfahren gemäss Anspruch 1, dadurch gekennzeichnet, dass man 9(S)-[[Hydroxy-(2-phenäthyl)phosphinyl]-methyl]-octahydro-10 -oxo-6H-pydridazo[1,2-a][1,2]diazepin-1(S)-carbonsäure herstellt.

17. Verfahren gemäss Anspruch 1, dadurch gekennzeichnet, dass man 9(S)-[Hydroxy-(4-phenylbutyl)phosphinyl]-octahydro-10-oxo-6H -pyridazo[1,2-a][1,2]diazepin-1(S)-carbonsäure herstellt.

18. Verwendung einer Verbindung gemäß Formel I gemäß einem der vorgehenden Ansprüche zur Herstellung eines Antihypertensivums.

***

0211220

Europäisches Patentamt

# EUROPÄISCHER RECHERCHENBERICHT

Nummer der Anmeldung

EP 86 10 8736

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 094 095 (HOFFMANN-LA ROCHE)<br>* Ansprüche 1, 29, 30 *<br>--- | 1,19, 20 | C 07 F 9/65<br>A 61 K 31/675<br>C 07 D 487/04 //<br>(C 07 D 487/04<br>C 07 D 243:00<br>C 07 D 237:00 ) |
| X,P | EP-A-0 172 552 (HOFFMANN-LA ROCHE)<br>* Anspruch 14 in Verbindung mit Spalte 7, Zeilen 38-43 *<br>----- | 19 | |
| | | | RECHERCHIERTE SACHGEBIETE (Int. Cl.4) |
| | | | C 07 F 9/65<br>A 61 K 31/675 |

Der vorliegende Recherchenbericht wurde für alle Patentansprüche erstellt

| Recherchenort | Abschlußdatum der Recherche | Prüfer |
|---|---|---|
| BERLIN | 17-09-1986 | KAPTEYN H G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82